# EUROPEAN PATENT SPECIFICATION

(11) **EP 1 656 141 B1**
(45) Date of publication and mention of the grant of the patent: **14.04.2010**
(21) Application number: 04767944.4
(22) Date of filing: 29.07.2004
(51) Int. Cl.: A61K 31/4439, A61K 31/403, C07D 401/04, C07D 209/86, C07D 209/88, A61P 25/28

(54) **TREATMENT FOR ALZHEIMER S DISEASE AND RELATED CONDITIONS**
BEHANDLUNG VON ALZHEIMER-KRANKHEIT UND VERWANDTEN ZUSTÄNDEN
PROCEDE POUR TRAITER LA MALADIE D'ALZHEIMER ET DES PATHOLOGIES LIEES

(30) Priority: 07.08.2003 GB 0318552; 08.04.2004 GB 0408100
(43) Date of publication of application: 17.05.2006
(73) Proprietor: MERCK SHARP & DOHME LTD., Hoddesdon, Hertfordshire EN11 9BU (GB)
(72) Inventor: BEHER, Dirk, Harlow Essex CM20 2QR (GB); BETTATI, Michela, Harlow Essex CM20 2QR (GB); CHECKSFIELD, Graham David, Harlow Essex CM20 2QR (GB); CHURCHER, Ian, Harlow Essex CM20 2QR (GB); DOUGHTY, Victoria Alexandra, Harlow Essex CM20 2QR (GB); OAKLEY, Paul Joseph, Harlow Essex CM20 2QR (GB); QUDDUS, Abdul, Harlow Essex CM20 2QR (GB); TEALL, Martin Richard, Harlow Essex CM20 2QR (GB); WRIGLEY, Jonathan David, Harlow Essex CM20 2QR (GB)
(74) Representative: Buchan, Gavin MacNicol
(86) International application number: PCT/GB2004/003286
(87) International publication number: WO 2005/013985

(56) References cited:
- EP-A- 0 234 708
- EP-A- 0 300 676
- EP-A- 0 307 077
- EP-A- 1 193 260
- WO-A-00/06560
- WO-A-02/08186
- WO-A-02/48150
- WO-A-99/25340
- WO-A-03/062200

## Description

This invention relates to methods and materials for use in therapeutic treatment of the human body. In particular, it provides methods of treating diseases associated with the deposition of β-amyloid peptide in the brain, such as Alzheimer's disease, or of preventing or delaying the onset of dementia associated with such diseases.

Alzheimer's disease (AD) is the most prevalent form of dementia. Its diagnosis is described in the Diagnostic and Statistical Manual of Mental Disorders, 4th ed., published by the American Psychiatric Association (DSM-IV). It is a neurodegenerative disorder, clinically characterized by progressive loss of memory and general cognitive function, and pathologically characterized by the deposition of extracellular proteinaceous plaques in the cortical and associative brain regions of sufferers. These plaques mainly comprise fibrillar aggregates of β-amyloid peptide (Aβ). Aβ is formed from amyloid precursor protein (APP) via separate intracellular proteolytic events involving the enzymes β-secretase and γ-secretase. Variability in the site of the proteolysis mediated by γ-secretase results in Aβ of varying chain length, e.g. Aβ(1-38), Aβ(1-40) and Aβ(1-42). N-terminal truncations such as Aβ(4-42) are also found in the brain, possibly as a result of variability in the site of proteolysis mediated by β-secretase. For the sake of convenience, expressions such as "Aβ(1-40)" and "Aβ(1-42)" as used herein are inclusive of such N-terminal truncated variants. After secretion into the extracellular medium, the initially-soluble Aβ forms aggregates which ultimately result in the insoluble deposits and dense neuritic plaques which are the pathological characteristics of AD.

Other dementing conditions associated with deposition of Aβ in the brain include cerebral amyloid angiopathy, hereditary cerebral haemorrhage with amyloidosis, Dutch-type (HCHWA-D), multi-infarct dementia, dementia pugilistica and Down syndrome.

Various interventions in the plaque-forming process have been proposed as therapeutic treatments for AD (see, for example, Hardy and Selkoe, Science, 297 (2002), 353-6). One such method of treatment that has been proposed is that of blocking or attenuating the production of Aβ for example by inhibition of β- or γ-secretase. It has also been reported that inhibition of glycogen synthase kinase-3 (GSK-3), in particular inhibition of GSK-3α, can block the production of Aβ (see Phiel et al, Nature, 423 (2003), 435-9).

Other proposed methods of treatment include administering a compound which blocks the aggregation of Aβ, and administering an antibody which selectively binds to Aβ.

An alternative mode of treatment is that of modulation of the action of γ-secretase so as to selectively attenuate the production of Aβ(1-42). This results in preferential secretion of the shorter chain isoforms of Aβ, which are believed to have a reduced propensity for self-aggregation and plaque formation, and hence are more easily cleared from the brain, and/or are less neurotoxic. Compounds showing this effect include certain non-steroidal antiinflammatory drugs (NSAIDs) and their analogues (see WO 01/78721 and US 2002/0128319 and Weggen et al Nature, 414 (2001) 212-16; Morihara et al, J. Neurochem., 83 (2002), 1009-12; and Takahashi et al, J. Biol. Chem., 278 (2003), 18644-70). Compounds which modulate the activity of PPARα and/or PPARδ are also reported to have the effect of lowering Aβ(1-42) (WO 02/100836). NSAID derivatives capable of releasing nitric oxide have been reported to show improved anti-neuroinflammatory effects and/or to reduce intracerebral Aβ deposition in animal models (WO 02/092072; Jantzen et al, J. Neuroscience, 22 (2002), 226-54).

EP 0234708, EP 0307077 and EP 0300676 disclose tetrahydrocarbazole 1-alkanoic acids which act as prostaglandin and thromboxane antagonists and are said to be useful in treating asthma, diarrhea, hypertension, angina, platelet aggregation, cerebral spasm, premature labour, spontaneous abortion and dysmenorrhea, as cytoprotective agents, and in limiting cyclosporine-induced nephrotoxicity. There is no disclosure or suggestion of any effect on the secretion of Aβ, or of any utility in the treatment or prevention of AD or any other disorders associated with deposition of Aβ in the brain.

WO0248150, EP 1193260, WO 9925340, WO 0006560 disclose various tricyclic compounds useful in treating AD or related disorders.

WHO 01/79169, WO 02/08186 and WO 03/062200 disclosed various cycloalkano-indoles as prostaglandin receptor antagonists, but again there is no disclosure of utility in treating AD or related disorders.

It has now been found that certain tetrahydrocarbazole 1-alkanoic acids and related compounds have the desirable property of selectively inhibiting production of Aβ(1-42).

According to the present invention there is provided the use, for the manufacture of a medicament for treatment or prevention of a disease associated with the deposition of β-amyloid in the brain, of a compound of formula I: 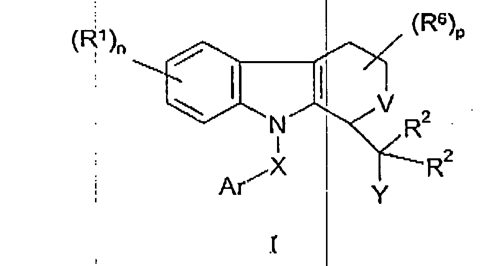
wherein V represents a bond, CH₂ or CH₂CH₂;
X represents CHR³ where R³ is H or a hydrocarbon group containing up to 10 carbon atoms which is optionally substituted with halogen, CF₃, C₁₋₄alkoxy or C₁₋₄alkylthio;
Y represents CO₂H;
Ar represents phenyl which optionally bears up to 3 substituents independently selected from hydrocarbon groups of up to 6 carbon atoms and (CH₂)ₘ-Z where m is 0, 1 or 2 and Z represents halogen, N₃, CN, CF₃, OCF₃, OR⁴, S(O)ₜR⁴ where t is 0, 1 or 2, CO₂R⁴, tetrazole, N(R⁴)₂, NHCOR⁵, NHCON(R⁴)₂, CON(R⁴)₂, SO₂N(R⁴)₂, NHSO₂R⁵, COR⁵, or OCOR⁵;
n is 0, 1, 2 or 3;
each R¹ is independently selected from nonaromatic hydrocarbon groups of up to 6 carbon atoms and (CH₂)_{q}-W where q is 0, 1 or 2 and W represents halogen, CN, CF₃ OR⁴, N(R⁴)₂, S(O)ₜR⁴ where t is 0, 1 or 2, CO₂R⁴, tetrazole, CON(R⁴)₂, SO₂N(R⁴)₂, COR⁵, OCOR⁵ or phenyl or heteroaryl either of which optionally bears up to 3 substituents selected from halogen, CF₃, OCF₃, CN, OH, C₁₋₄-alkyl, C₁₋₄alkoxy, C₁₋₄alkylthio or C₁₋₄alkoxycarbonyl;
each R² is independently H or C₁₋₄alkyl; or one R² group together with an R⁶ group attached at the same ring position as the -C(R²)₂-Y moiety completes a spiro-linked hydrocarbon ring of 3-6 members;
R⁴ represents H or hydrocarbon group of up to 7 carbon atoms, optionally substituted with halogen, CN, CF₃, OH, C₁₋₄alkoxy or C₁₋₄alkoxycarbonyl; or two R⁴ groups attached to the same nitrogen atom may complete a 5- or 6-membered heterocyclic ring;
R⁵ represents R⁴ that is other than H;
p is 0, 1 or 2; and
R⁶ represents C₁₋₆alkyl, C₂₋₆alkenyl or phenyl, benzyl or heteroaryl, said phenyl, benzyl or heteroaryl optionally bearing up to 3 substituents selected from halogen, CN, CF₃, OCF₃, OR⁴ CO₂R⁴, COR⁵, and C₁₋₄alkyl; or an R⁶ group together with an R² group may complete a spiro-linked hydrocarbon ring as defined previously;
or a pharmaceutically acceptable salt thereof.

In a particular embodiment of the invention, each R² is independently H or C₁-₄alkyl.
In a sub-embodiment, V represents CH₂;
X represents CHR³ where R³ is H or C₁₋₆alkyl;
Ar represents phenyl which optionally bears up to 3 substituents independently selected from nonaromatic hydrocarbon groups of up to 6 carbon atoms and (CH₂)ₘ-Z where m is 0, 1 or 2 and Z represents halogen, N₃, CN, CF₃, OR⁴, S(O)₁R¹ where t is 0, 1 or 2, CO₂R⁴, tetrazole, N(R⁴)₂, NHCOR⁵, NHCON(R⁴)₂, CON(R⁴)₂, SO₂N(R⁴)₂, NHSO₂R⁵, COR⁵, or OCOR⁵;
each R¹ is independently selected from nonaromatic hydrocarbon groups of up to 6 carbon atoms and (CH₂)_{q}-W where q is 0,1 or 2 and W represents halogen, CN, CF₃, OR⁴, S(O)ₜR⁴ where t is 0,1 or 2, CO₂R⁴, tetrazole, CON(R⁴)₂, SO₂N(R⁴)₂, COR⁵, OCOR⁵ or phenyl which is optionally substituted with halogen, CF₃, CN, OH, C₁₋₄alkyl, C₁₋₄alkoxy or C₁₋₄alkoxycarbonyl;
R⁴ represents H or a hydrocarbon group of up to 7 carbon atoms, optionally substituted with halogen, CN, CF₃, OH, C₁₋₄alkoxy or C₁₋₄alkoxycarbonyl;
R⁶ represents C₁₋₆alkyl, C₂₋₆alkenyl or phenyl or benzyl, said phenyl or benzyl optionally bearing up to 3 substituents selected from halogen, CN, CF₃, OR⁴, CO₂R⁴, COR⁵, OCOR⁵ and C₁₋₄alkyl;
and Y, n, R², R⁵ and p are as defined previously.
The disease associated with deposition of Aβ in the brain is typically Alzheimer's disease (AD), cerebral amyloid angiopathy, multi-infarct dementia, dementia pugilistica or Down syndrome, preferably AD.

In a second aspect, the invention provides the use of a compound of Formula I as defined above, or a pharmaceutically acceptable salt thereof, in the manufacture of a medicament for treating, preventing or delaying the onset of dementia associated with Alzheimer's disease, cerebral amyloid angiopathy, HCHWA-D, multi-infarct dementia, dementia pugilistica or Down syndrome.

The invention also provides a method of treating or preventing a disease associated with deposition of Aβ in the brain comprising administering to a patient in need thereof a therapeutically effective amount of a compound of Formula I as defined above or a pharmaceutically acceptable salt thereof.

In a further aspect, the invention provides a method of treating, preventing or delaying the onset of dementia associated with Alzheimer's disease, cerebral amyloid angiopathy, HCHWA-D, multi-infarct dementia, dementia pugilistica or Down syndrome comprising administering to a patient in need thereof a therapeutically effective amount of a compound of Formula I as defined above or a pharmaceutically acceptable salt thereof.

The compounds of Formula I modulate the action of γ-secretase so as to selectively attenuate production of the (1-42) isoform of Aβ without significantly lowering production of the shorter chain isoforms such as Aβ(1-40). This results in secretion of Aβ which has less tendency to self-aggregate and form insoluble deposits, is more easily cleared from the brain, and/or is less neurotoxic. Therefore, a further aspect of the invention provides a method for retarding, arresting or preventing the accumulation of Aβ in the brain comprising administering to a subject in need thereof a therapeutically effective amount of a compound of Formula I as defined above or a pharmaceutically acceptable salt thereof.

Because the compounds of formula I modulate the activity of γ-secretase, as opposed to suppressing said activity, it is believed that the therapeutic benefits described above will be obtained with a reduced risk of side effects, e.g. those that might arise from a disruption of other activities mediated by γ-secretase, such as the Notch signalling process.

In one embodiment of the invention, the compound of Formula I is administered to a patient suffering from AD, cerebral amyloid angiopathy, HCHWA-D, multi-infarct dementia, dementia pugilistica or Down syndrome, preferably AD.

In an alternative embodiment of the invention, the compound of Formula I is administered to a patient suffering from mild cognitive impairment or age-related cognitive decline. A favourable outcome of such treatment is prevention or delay of the onset of AD. Age-related cognitive decline and mild cognitive impairment (MCI) are conditions in which a memory deficit is present, but other diagnostic criteria for dementia are absent (Santacruz and Swagerty, American Family Physician, 63 (2001), 703-13). (See also "The ICD-10 Classification of Mental and Behavioural Disorders", Geneva: World Health Organisation, 1992, 64-5). As used herein, "age-related cognitive decline" implies a decline of at least six months' duration in at least one of: memory and learning; attention and concentration; thinking; language; and visuospatial functioning and a score of more than one standard deviation below the norm on standardized neuropsychologic testing such as the MMSE. In particular, there may be a progressive decline in memory. In the more severe condition MCI, the degree of memory impairment is outside the range considered normal for the age of the patient but AD is not present. The differential diagnosis of MCI and mild AD is described by Petersen et al., Arch. Neurol., 56 (1999), 303-8. Further information on the differential diagnosis of MCI is provided by Knopman et al, Mayo Clinic Proceedings, 78 (2003), 1290-1308. In a study of elderly subjects, Tuokko et al (Arch, Neurol., 60 (2003) 577-82) found that those exhibiting MCI at the outset had a three-fold increased risk of developing dementia within 5 years.

Grundman et al (J. Mol. Neurosci., 19 (2002), 23-28) report that lower baseline hippocampal volume in MCI patients is a prognostic indicator for subsequent AD. Similarly, Andreasen et al (Acta Neurol. Scand, 107 (2003) 47-51) report that high CSF levels of total tau, high CSF levels of phospho-tau and lowered CSF levels of Aβ42 are all associated with increased risk of progression from MCI to AD.

Within this embodiment, the compound of Formula I is advantageously administered to patients who suffer impaired memory function but do not exhibit symptoms of dementia. Such impairment of memory function typically is not attributable to systemic or cerebral disease, such as stroke or metabolic disorders caused by pituitary dysfunction. Such patients may be in particular people aged 55 or over, especially people aged 60 or over, and preferably people aged 65 or over. Such patients may have normal patterns and levels of growth hormone secretion for their age. However, such patients may possess one or more additional risk factors for developing Alzheimer's disease. Such factors include a family history of the disease; a genetic predisposition to the disease; elevated serum cholesterol; and adult-onset diabetes mellitus.

In a particular embodiment of the invention, the compound of Formula I is administered to a patient suffering from age-related cognitive decline or MCI who additionally possesses one or more risk factors for developing AD selected from: a family history of the disease; a genetic predisposition to the disease; elevated serum cholesterol; adult-onset diabetes mellitus; elevated baseline hippocampal volume; elevated CSF levels of total tau; elevated CSF levels of phospho-tau; and lowered CSF levels of Aβ(1-42).

A genetic predisposition (especially towards early onset AD) can arise from point mutations in one or more of a number of genes, including the APP, presenilin-1 and presenilin-2 genes. Also, subjects who are homozygous for the ε4 isoform of the apolipoprotein E gene are at greater risk of developing AD.

The patient's degree of cognitive decline or impairment is advantageously assessed at regular intervals before, during and/or after a course of treatment in accordance with the invention, so that changes therein may be detected, e.g. the slowing or halting of cognitive decline. A variety of neuropsychological tests are known in the art for this purpose, such as the Mini-Mental State Examination (MMSE) with norms adjusted for age and education (Folstein et al. , J. Psych. Res., 12 (1975), 196-198, Anthony et al., Psychological Med., 12 (1982), 397-408; Cockrell et al., Psychopharmacology, 24 (1988), 689-692; Crum et al., J. Am. Med. Assoc'n. 18 (1993), 2386-2391). The MMSE is a brief, quantitative measure of cognitive status in adults. It can be used to screen for cognitive decline or impairment, to estimate the severity of cognitive decline or impairment at a given point in time, to follow the course of cognitive changes in an individual over time, and to document an individual's response to treatment. Another suitable test is the Alzheimer Disease Assessment Scale (ADAS), in particular the cognitive element thereof (ADAS-cog) (See Rosen et al., Am. J. Psychiatry, 141 (1984), 1356-64).

Where a variable occurs more than once in formula I or in a substituent thereof, the individual occurrences of that variable are independent of each other, unless otherwise specified.

As used herein, the expression "hydrocarbon group" refers to groups consisting solely of carbon and hydrogen atoms. Such groups may comprise linear, branched or cyclic structures, singly or in any combination consistent with the indicated maximum number of carbon atoms, and may be saturated or unsaturated, including aromatic when the indicated maximum number of carbon atoms so permits unless otherwise indicated.

As used herein, the expression "C₁₋ₓ₋alkyl" where x is an integer greater than 1 refers to straight-chained and branched alkyl groups wherein the number of constituent carbon atoms is in the range 1 to x. Particular alkyl groups are methyl, ethyl, n-propyl, isopropyl and t-butyl. Derived expressions such as "C₂₋₆alkenyl", "hydroxyC₁-₆alkyl", "heteroarylC₁₋₆alkyl", "C₂₋₆alkynyl" and "C₁₋₆alkoxy" are to be construed in an analogous manner. Most suitably, the number of carbon atoms in such groups is not more than 6.

The term "halogen" as used herein includes fluorine, chlorine, bromine and iodine, of which fluorine and chlorine are preferred.

The term "heteroaryl" as used herein means a cyclic or polycyclic system of up to 10 ring atoms selected from C, N, O and S, wherein at least one of the constituent rings is aromatic and wherein at least one atom of the aromatic ring is other than carbon. Preferably not more than 3 ring atoms are other than carbon. Examples of heteroaryl groups include pyridinyl, pyridazinyl, pyrimidinyl, pyrazinyl, pyrrolyl, furyl, thienyl, pyrazolyl, oxazolyl, isoxazolyl, thiazolyl, isothiazolyl, imidazolyl, oxadiazolyl, triazolyl and thiadiazolyl groups and benzo-fused analogues thereof Further examples of suitable heteroaryl ring systems include 1,2,4-triazine, 1,3,5-triazine, 1,2,3,4-tetrahydroquinoline and 1,2,3,4-tetrahydroisoquinoline. Monocyclic 5- or 6-membered systems are preferred, especially pyridine or thiophene.

For use in medicine, the compounds of formula I may be in the form of pharmaceutically acceptable salts. Other salts may, however, be useful in, the preparation of the compounds of formula I or of their pharmaceutically acceptable salts. Suitable pharmaceutically acceptable salts of the compounds of this invention include acid addition salts which may, for examples, be formed by mixing a solution of the compound according to the invention with a solution of a pharmaceutically acceptable acid such as hydrochloric acid, sulphuric acid, methanesulphonic acid, benzenesulphonic acid, fumaric acid, maleic acid, succinic acid, acetic acid, benzoic acid, oxalic acid, citric acid, tartaric acid, carbonic acid or phosphoric acid. Alternatively, where the compound of the invention carries an acidic moiety, a pharmaceutically acceptable salt may be formed by neutralisation of said acidic moiety with a suitable base. Examples of pharmaceutically acceptable salts thus formed include alkali metal salts such as sodium or potassium salts; ammonium salts; alkaline earth metal salts such as calcium or magnesium salts; and salts formed with suitable organic bases, such as amino salts (including pyridinium salts) and quaternary ammonium salts.

Where the compound according to the invention have at least one asymmetric centre, they may accordingly exist as enantiomers. Where the compounds according to the invention possess two or more asymmetric centres, they may additionally exist as diastereoisomers. It is to be understood that all such isomers and mixtures thereof in any proportion are encompasses within the or the present invention.

In formula I, V represents a bond, CH₂ or CH₂CH₂. In a particular embodiment V represents CH₂ and the compounds of formula I are 9-substituted-1,2,3,4-tetrahydrocarbazol-1-yl acetic acid derivatives.

X represents CHR³ where R³ represents H or a hydrocarbon group of up to 10 carbon atoms which is optionally substituted as defined previously. Suitable identities for R³ include H; alkyl (especially C₁-₆alkyl such as methyl, ethyl, n-propyl, isopropyl, 2-methylpropyl, n-butyl, 3-methylbutyl and 4-methylpentyl); substituted alkyl (such as methoxymethyl, methylthiomethyl and 3,3,3-trifluoropropyl); alkenyl (such as allyl); cycloalkyl (especiallyC₃₋₆cycloalkyl such as cyclopropyl, cyclopentyl and cyclohexyl); cycloalkylalkyl (such as cyclopropylmethyl and cyclohexylethyl); aryl (such as phenyl and 4-trifluoromethylphenyl) and arylalkyl (such as benzyl). In a particular embodiment, R³ is an optionally-substituted hydrocarbon group of 2 to 10 carbon atoms, preferably 2 to 6 carbon atoms, and in particular an alkyl group of 2 to 6 carbon atoms. Preferred identities for X include CH₂, CHCH₃, CHCH₂CH₃ and CHCH₂CH₂CH₃.

Y represents CO₂H.

Ar represents phenyl which is optionally substituted as defined previously. Phenyl groups represented by Ar optionally bear up to 3 substituents as defined previously. When said substituents comprise a group represented by (CH₂)ₘ-Z, m is preferably 0 or 1, most preferably 0. When Ar represents mono-substituted phenyl, the substituents aptly occupies the 4-position. Examples of suitable substituents include halogen (especially Cl and F), N₃, CF₃, OCF₃, OH, OMe, SMe, NHCOMe, SO₂Me, CO₂H, CO₂Me, C₁₋₄alkyl (such as methyl, ethyl, n-propyl and isopropyl), CON(Me)₂, COMe, SO₂N(Me)₂, NHSO₂Me and NHCONHMc. Preferred substituents include Cl, F, N₃, OCF₃, CF₃ and OMe.

Specific examples of groups represented by Air include phenyl, 4-chlorophenyl, 4-trifluoromethylphenyl, 4-fluorophenyl, 4-azidophenyl, 4-methoxyphenyl, 4-trifluoromethoxypheny, 2,4-bis(trifluoromethyl)phenyl, 3,4-dichlorophenyl, 2,4-dichlorophenyl, 2,4,6-trifluorophenyl and 4-iodophenyl, of which 4-chlorophenyl and 4-trifluoromethylphenyl are particularly preferred.

In Formula I, n is 0,1, 2, or 3, but is preferably 0, 1 or 2, most preferably 1 or 2. Each R¹ group is independently selected from non-aromatic hydrocarbon of up to 6 carbon atoms and (CH₂)_{q}-W where q is 0, 1 or 2 and W is as defined previously. Preferably, q is 0 or 1, and most preferably q is 0 Non-aromatic hydrocarbon groups represented by R¹ are very aptly linear or branched C₁₋₆alkyl groups such as methyl, ethyl, n-propyl, isopropyl and t-butyl, of which methyl, isopropyl, n-butyl and t-butyl arc particularly preferred, or C₃₋₆cycloalkyl, such as cyclopropyl and cyclohcxyl. Examples of groups represented by W include halogen (especially F, Cl and Br), CN, CF₃, OMe, SMe, S(O)Me, SO₂Me N(R⁴)₂ (in particular where the R⁴ groups complete a heterocyclic ring such as pyrrolidine, piperidine or morpholine) and optionally-substituted phenyl or heteroaryl. Preferred examples of heteroaryl groups represented by W include pyridyl (especially 3-pyridyl) and thiophene (e.g. 3-thienyl). Preferred examples of substituted phenyl groups represented by W include 4-fluorophenyl, 3,4-dichlorophenyl, 3-methylthiophenyl, 2,5-dimethylphenyl and 3-trifluoromethoxyphenyl. Preferred identities for R¹ include methyl, ethyl, isopropyl, n-butyl, t-butyl, cyclopropyl, Br, Cl, F, CN, CF₃, OCH₃, QCF₃, SCH₃, morpholin-1-yl, 4-fluorophenyl, 3,4-dichlorophenyl, 3-methylthiophenyl, 2,5-dimethylphenyl and 3-trifluoromethoxyphenyl.

Each R² is independently H or C₁₋₄alkyl such as methyl, ethyl or propyl. Preferably one R² is H and the other is H or alkyl. Most preferably, both R² groups are H. Alternatively, when p is not zero, one R² group together with an R⁶ group attached at the same ring position as the -C(R²)₂-Y moiety completes a spiro-linked hydrocarbon ring of 3-6 members, e.g. cyclopropyl.

When present, R⁶ represents linear or branched C₁₋₆alkyl (preferably C₁₋₄alkyl) such as methyl, ethyl, n-propyl, isopropyl or t-butyl, C₂₋₆ alkenyl such as vinyl or allyl, or phenyl, heteroaryl or benzyl which is optionally substituted as defined previously. Preferred substituents include halogen (especially Cl or F), OCH₃, OCF₃, CF₃ and C₁-₄alkyl (such as methyl). A preferred heteroaryl group is pyridyl, especially 3-pyridyl. Examples of groups represented by R⁶ include methyl, isopropyl, vinyl, 3-pyridyl, phenyl, 4-chlorophenyl, 3-fluorophenyl, 4-fluorophenyl, 4-fluoro-3-methylphenyl, 4-methoxyphenyl, 3,4-dichlorophenyl, 3,4-difluorophenyl and 2,5-dimethylphenyl. An R⁶ group may be attached at any available position of the ring, including the carbon atom bearing the -C(R²)₂-Y moiety and any carbon atom included in V. Where two R⁶ groups are present, they may be the same or different and may be attached to the same or different ring positions. When p is 2, preferably not more than one of the R⁶ groups is optionally-substituted phenyl, heteroaryl or benzyl. Alternatively, an R⁶ group may combine with an R² to complete a spiro-linked ring as defined previously.

Specific examples of compounds suitable for use in the invention include 9-substituted-1,2,3,4-tetrahydrocarbazol-1-yl acetic acid derivatives of formula I in which V is CH₂ and Y is CO₂H and the remaining variables are as indicated in the following table.

**Table 1**

| **Compound** | **(R¹)ₙ** | **R²,R²** | **(R⁶)ₚ** | **X** | **Ar** |
|---|---|---|---|---|---|
| 1 | 6-OMe | H,H | p=0 | CH₂ | 4-Cl-Ph |
| 2 | 6-F | H,H | p=0 | CH₂ | 4-Cl-Ph |
| 3 | 6-F | H,H | p=0 | CH₂ | Ph |
| 4 | 6-F | H,H | p=0 | CH₂ | 4-McO-Ph |
| 5 | 6-F | H,H | p=0 | CH₂ | 3,4-di-Cl-Ph |
| 6 | 6-Me | H,H | p=0 | CH(Me) | 4-CF₃-Ph |
| 7 | 6-F | H,H | p=0 | CH(Mc) | Ph |
| 8 | H | H,H | p=0 | CH₂ | 4-Cl-Ph |
| 9 | 6-Cl | H,H | p=0 | CH₂ | 4-Cl-Ph |
| 10 | 5,7-di-Cl | H,H | p=0 | CH₂ | 4-Cl-Ph |
| 11 | 6-F | H,Me | p=0 | CH₂ | 4-Cl-Ph |
| 12 | 6-F | H,H | 3-Me | CH₂ | 4-Cl-Ph |
| 13 | 6-SMe | H,H | p=0 | CH₂ | 4-Cl-Ph |
| 14 | 6-isopropyl | H,H | p=0 | CH₂ | 4-CF₃-Ph |
| 15 | 6-isopropyl | H,H | p=0 | CH(Me) | 4-CF₃-Ph |
| 16 | 6-isopropyl | H,H | p=0 | CH₂ | 4-Cl-Ph |
| 18 | 6-Me, 8-F | H,H | p=0 | CH₂ | 4-CF₃-Ph |
| 19 | 6-Me, 8-F | H,H | p=0 | CH(Me) | 4-CF₃-Ph |
| 20 | 6-isopropyl | H,H | p=0 | CH₂ | 2,4-di-Cl-Ph |
| 21 | 6-isopropyl | H,H | p=0 | CH₂ | 4-I-Ph |
| 22 | 6-isopropyl | H,H | p=0 | CH₂ | 2,4,6-tri-F-Ph |
| 23 | 6,8-di-Cl | H,H | p=0 | CH₂ | 4-CF₃-Ph |
| 24 | 6,8-di-Me | H,H | p=0 | CH₂ | 4-CF₃-Ph |
| 25 | 6,8-di-Br | H,H | p=0 | CH₂ | 4-CF₃-Ph |
| 26 | 6,8-di-Br | H,H | p=0 | CH(Me) | 4-CF₃-Ph |
| 27 | 8-Cl | H,H | p=0 | CH₂ | 4-CF₃-Ph |
| 28 | 6-isopropyl | H,H | p=0 | CH₂ | 4-F-Ph |
| 29 | 6-isopropyl | H,H | p=0 | CH₂ | 4-N₃-Ph |
| 30 | 6-isopropyl | H,H | 3-Ph | CH₂ | 4-CF₃-Ph |
| 31 | 6-isopropyl | H,H | p=0 | CH(Me) | 2,4,6-tri-F-Ph |
| 32 | 6-isopropyl | H,H | 4-(4-F-Ph) | CH₂ | 4-CF₃-Ph |
| 33 | 6-isopropyl | H,H | p=0 | CH(Et) | 4-CF₃-Ph |
| 34 | 6-isopropyl-8-Br | H,H | p=0 | CH₂ | 4-CF₃-Ph |
| 35 | 6-isopropyl | H,H | 1-Me | CH₂ | 4-CF₃-Ph |
| 36 | 5-(4-F-Ph) | H,H | p=0 | CH₂ | 4-CF₃-Ph |
| 37 | 6-isopropyl | H,H | 1-Me, 4-vinyl | CH₂ | 4-CF₃-Ph |

A subset of the compounds of Formula I is defined by Formula II: 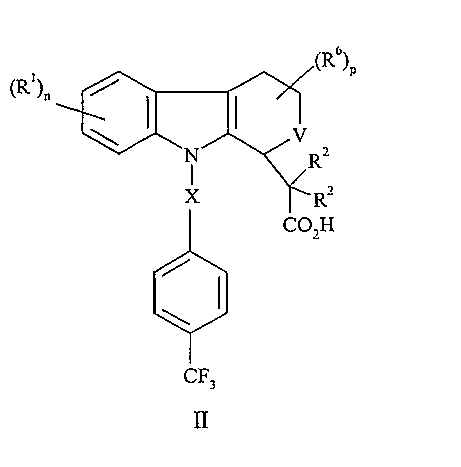 wherein V, X, n, p, R¹, R² and R⁶ have the same definitions and preferred identities as before.

With the exception of the compound in which V is CH₂, X is CH₂, p is 0, each R² is H, and (R¹)ₙ represents 6,8-difluoro, the compounds of Formula II in which V is CH₂ or CH₂CH₂ and the pharmaceutically acceptable salts thereof are believed to be novel, and hence constitute a further aspect of the invention. The invention further extends to pharmaceutical compositions comprising, in a pharmaceutically acceptable carrier, a compound of Formula II wherein V is CH₂ or CH₂CH₂ or a pharmaceutically acceptable salt thereof, with the exception of the compound in which V is CH₂, X is CH₂, p is 0, each R² is H, end (R¹)ₙ represents 6,8-difluoro.

In Formula II, X is CHR³, in particular, CH₂, CH(Me), CH(Et) or CH(Pr). Particularly preferred examples include the compounds:
{6-isopropyl-9-[1-(4-trifluoromethylphenyl)ethyl]-2,3,4,9-tetrahydro-1H-carbazol-1-yl}-acetic acid; and
{6-isopropyl-9-[1-(4-trifluoromethylphenyl)propyl]-2,3,4,9-tetrahydro-1H-carbazol-1-y1}-acetic acid; and
{6-isopropyl-9-[1-(4-triftuoromethylphenyl)butyl]-2,3,4,9-tetrahydro-1H-carbazol-1-yl}-acetic acid.

Compounds of Formula I in which p is 1 or 2 and at least one R⁶ represents C₂-₆alkenyl or phenyl, heteroaryl or benzyl which are optionally substituted as described previously are also novel, and said compounds, their pharmaceutically acceptable salts, and pharmaceutical compositions comprising them constitute a further aspect of the invention. In this context, V is preferably CH₂, p is preferable 1 any R⁶ is preferably optionally substituted phenyl, such as phenyl or 4-fluorophenyl, attached to the 3-position or the 4-position of the tetrahydrocarbazole ring.

A further novel subset of the compounds of Formula I is defined by formula III: 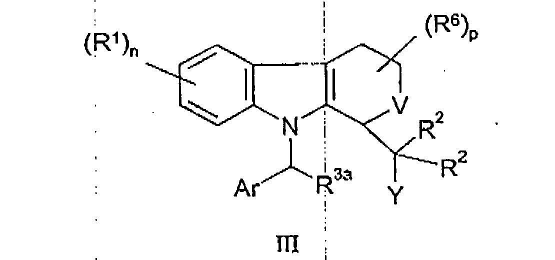
wherein R^{3a} represents a hydrocarbon group containing from 2 to 10 carbon atoms which is optionally substituted with halogen, CF₃, C₁₋₄alkoxy or C₁₋4alkylthio;
and V, Y, Ar, n, p, R¹, R² and R⁶ have the same definitions and preferred identities at before, with the proviso that R¹ does not represent SOR⁴ or SO₂R⁴.

Compounds of formula III and pharmaceutically acceptable salts thereof, constitute a Further aspect of the invention. The invention further extends to pharmaceutical compositions comprising a compound of formula III or a pharmaceutically acceptable salt thereof and a pharmaceutically acceptable carrier.

In the compounds of formula III, R^{3a} very aptly represents c₃₋₆alkyl, in particular n-propyl. V is very aptly CH₂. Ar is very aptly 4-trifluoromethylphenyl. Preferably at least one R² is H and most preferably both R² groups represent H.

Specific examples of compounds in accordance with formula III include 9-substituted-1,2,3,4-tetrahydrocarbazol-1-yl acetic acid derivatives in which V represents CH₂, Y represents CO₂H, Ar represents 4-trifluoromethylphenyl, each R² is H (unless otherwise indicated) and the other variables are as shown in table 2:

**Table 2**

| **Cpd.** | **(R¹)ₙ** | **(R⁶)ₙ** | **R^{3a}** |
|---|---|---|---|
| 33 | 6-isopropyl | p=0 | ethyl |
| 38 | 6-isopropyl | p=0 | propyl |
| 39 | 6,8-dichloro | p=0 | ethyl |
| 40 | 6-isopropyl | 4,4-di-Me | ethyl |
| 41 | 5-bromo | p=0 | n-pfopyi |
| 42 | 5-(3,4-di-Cl-Ph) | p=0 | ethyl |
| 43 | 6,8-dichloro | p=0 | n-propyl |
| 44 | 5,7-dichloro, | p=0 | n-propyl |
| 45 | 6-chloro | p=0 | n-propyl |
| 46 | 6-isopropyl | p=0 | 2-methylpropyl |
| 47 | 8-chloro | p=0 | n-propyl |
| 48 | n=0 | p=0 | n-propyl |
| 49 | 6-isopropyl | 4-(4-F-Ph) | n-propyl |
| 50 | 6-isopropyl | p=0 | 4-CF₃-Ph |
| 51 | 6-bromo | p=0 | n-propyl |
| 52 | 6-(3,4-di-Cl-Ph) | p=0 | n-propyl |
| 53 | 6,8-difluoro | p=0 | n-propyl |
| 54 | 6-isopropyl | p=0 | cyclohexyl |
| 55 | 6-isopropyl | p=0 | isopropyl |
| 56 | 6-isopropyl | p=0 | methoxymethyl |
| 57 | 5-(3,4-di-Cl-Ph) | p=0 | n-propyl |
| 58 | 6-n-butyl | p=0 | n-propyl |
| 59 | 8-Cl-6-isopropyl | p=0 | n-propyl |
| 60 | 5-(3-OCF₃-Ph) | p=0 | n-propyl |
| 61 | 5-OCF₃ | p=0 | n-propyl |
| 62 | 6-isopropyl | p=0 | n-butyl |
| 63 | 5-CN | p=0 | n-propyl |
| 64 | 6-isopropyl | p=0 | 3-methylbutyl |
| 65 | 5-(morpholin-1-yl) | p=0 | n-propyl |
| 66 | 6-isopropyl | p=0 | 3,3,3-trifluoropropyl |
| 67 | 6-cyclopropyl | p=0 | n-propyl |
| 68 | 7-cyclopropyl | p=0 | n-propyl |
| 69 | 7-bromo | p=0 | n-propyl |
| 70 | 5-cyclopropyl | p=0 | n-propyl |
| 71 | 6-CN | p=0 | n-propyl |
| 72 | 5-isopropyl | p=0 | n-propyl |
| 73 | 5-(3,4-di-Cl-Ph)-6-isopropyl | p=0 | n-propyl |
| 74 | 6-isopropyl | p=0 | methylthiomethyl |
| 75 | 6-t-butyl | p=0 | n-propyl |
| 76 | 6-isopropyl | p=0 | allyl |
| 77 | 6-isopropyl | p=0 | cyclohexylethyl |
| 78 | 6-isopropyl | p=0 | 4-methylpentyl |
| 79 | 6-isopropyl | 1-"propyl | n-propyl |
| 80 | 6-isopropyl | *1-spirocyclopropyl | n-propyl |

| | | | |
|---|---|---|---|
| * together with R² | | | |

Further examples of specific compounds in accordance with formula III include those of the following formula wherein the variables are as shown in table 3:

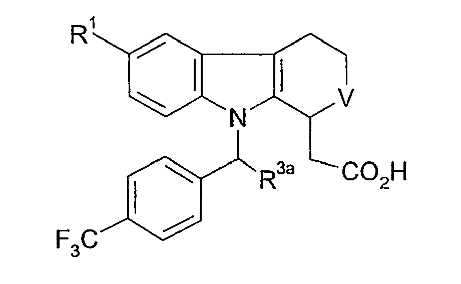

**Table 3**

| **Compound** | **V** | **R¹** | **R^{3a}** |
|---|---|---|---|
| 81 | bond | isopropyl | n-propyl |
| 82 | CH₂CH₂ | isopropyl | n-propyl |

It will be apparent to those skilled in the art that in formula III the carbon atom to which R^{3a} is attached and the carbon atom to which C(R²)₂-Y is attached are both chiral centres, and hence that the relevant compounds exist in at least two diastereomeric and at least four enantiomeric forms: 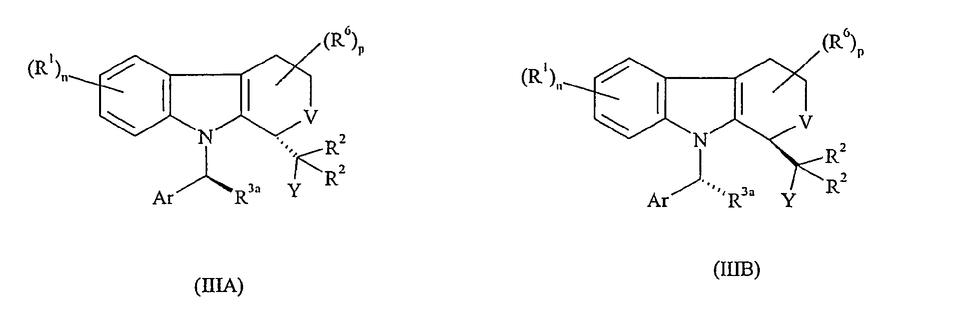 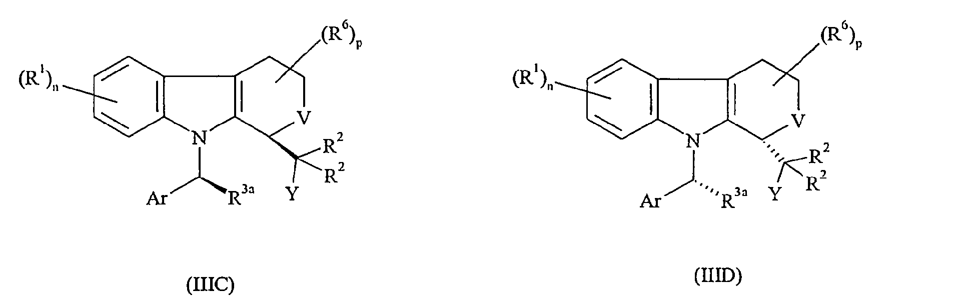 where V, Y, Ar, n, p, R¹, R², R^{3a} and R⁶ have the same meanings as before.

It is to be understood that all such isomers are included within the scope of the invention, as pure compounds or as mixtures of isomers in any proportion.

Compounds of Formula I in which X is SO₂ may be prepared by reaction of compounds (1) with ArSO₂Cl: 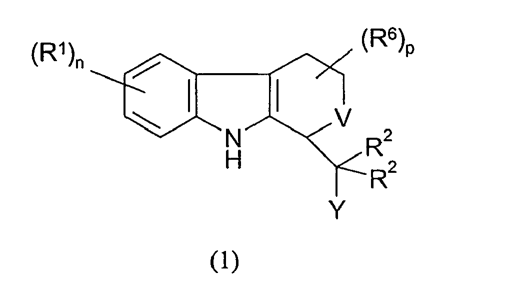 where V, Ar, Y, n, p, R¹, R² and R⁶ have the same meanings as before. The reaction takes place in the presence of a base such as triethylamine in an aprotic solvent.

Compounds of formula I in which X is CHR³ may be prepared by N-alkylation of compounds of formula (1) with ArCH(R³)-L where L is a leaving group such as Cl, Br, I, mesylate, tosylate or triflate, and Ar and R³ have the same meaning as before. The N-alkylation may be carried out by treating the compound of formula I with strong base such as sodium hydride or potassium t-butoxide in DMF at about 0°C, then adding ArCH(R³)-L and warming to ambient temperature.

Compounds of formula (1) may be prepared by the well-known Fischer indole synthesis route, involving condensation of a hydrazine (2) with a ketone (3): 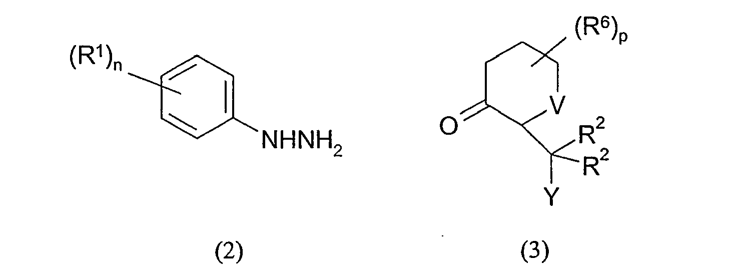 where V, Y, n, p, R¹, R² and R⁶ have the same meanings as before. The reaction may be carried out by refluxing in a lower alkanol.

An alternative route to compounds of formula (1) involves reaction of a ketone (3) with an iodoaniline (4): 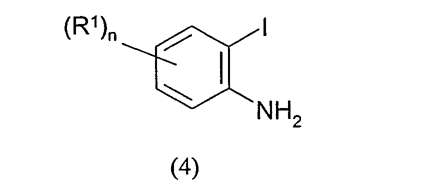 where n and R¹ have the same meaning as before. The reaction takes place in DMF solution in the presence of Si(OEt)₄ and an acid such as toluenesulphonic acid, followed by treatment with palladium acetate and Hunig's base.

Compounds of formula I in which X is CHR³ may also be prepared directly by the Fischer indole route using a hydrazine of formula (5) instead of the hydrazine of formula (2): 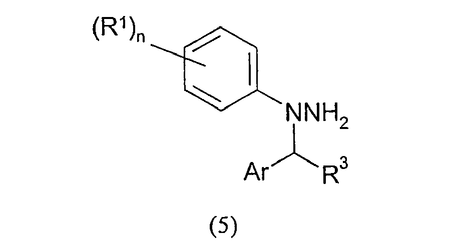 where Ar, n, R¹ and R³ have the same meaning as before. EP0234708 discloses detailed procedures for the Fischer indole route applied to ketones (3) in which V is CH₂. These procedures are equally applicable to ketones (3) in which V is a bond or CH₂CH₂.

A preferred route compounds of formula I in which p is 1 and R⁶ is attached as shown in compounds (9) below comprises oxidation of compounds (1) in which p is 0 to form ketones (6a), followed by treatment with ArSO₂Cl to give sulfonamides (6b): 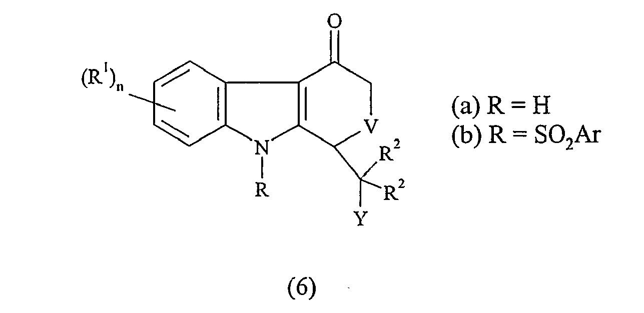 followed by conversion to the corresponding enol triflates (7): 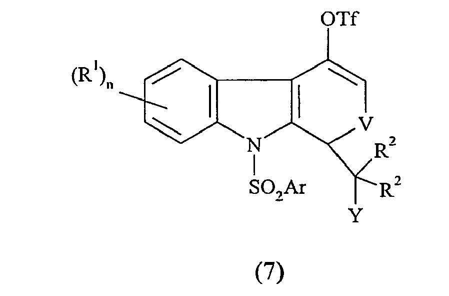 followed by treatment with R⁶-B(OH)₂ to give the compounds of formula (8): 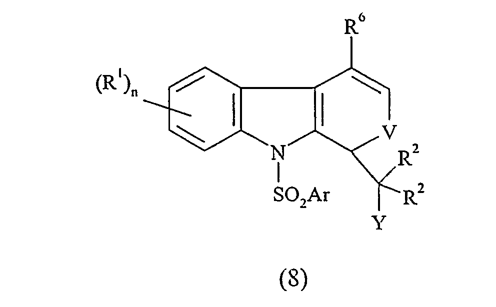 followed by hydrogenation to give the compounds of formula (9a): 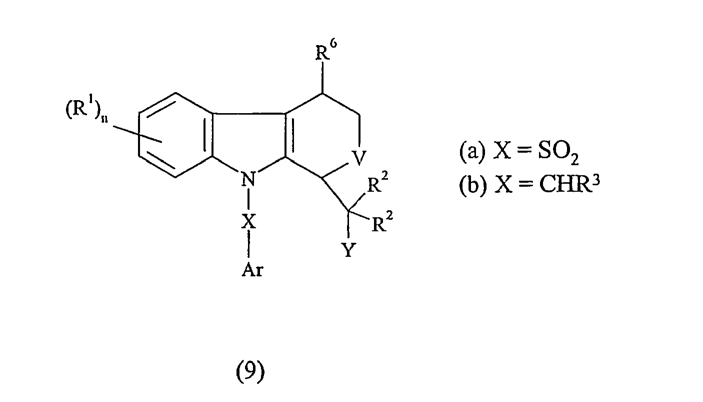 where Tf represents trifluoromethanesulfonyl (triflyl) and n, R¹, R², R⁶, V, Y and Ar have the same meanings as before.

In the above scheme, R⁶ very suitably represents phenyl or substituted phenyl, such as 4-fluorophenyl.

The oxidation to form ketones (6a) may be carried out using DDQ in aqueous THF at about 0°C, while the treatment with ArSO₂Cl may be carried out as described previously. Formation of triflates (7) takes place in THF at low temperature (e.g. -78°C) in the presence of strong base (such as lithium hexamethyldisilazide) and N-phenylbis(trifluoromethanesulfonimide). Treatment with R⁶-B(OH)₂ may be carried out in dioxan at about 80°C in the presence of potassium phosphate and Pd(PPh₃)ₐ. The hydrogenation may be carried out over a Pd/C catalyst in ethyl acetate.

The corresponding compounds (9b) (in which X represents CHR³) may be prepared by treatment of compounds (8) in which Ar is 4-methylphenyl with sodium amalgam and NaH₂PO₄, followed by N-alkylation of the detosylated product with ArCH(R³)-L, then hydrogenation as before, where L is a leaving group such as Cl, Br, I, mesylate, tosylate or triflate, and R³ has the same meaning as before. The treatment with sodium amalgam and NaH₂PO₄ may be carried out at ambient temperature in a THF - methanol mixture. The N-alkylation may be carried out by treating the detosylated product with sodium hydride in DMF at about 0°C, then adding ArCH(R³)-L and warming to ambient temperature.

During all of the chemical processes described above, a carboxylic acid group represented by Y is preferably protected as the methyl ester or ethyl ester, the free acid being regenerated by hydrolysis in a final step, e.g. using LiOH in aqueous THF.

Since the compounds of Formula I have at least one asymmetric centre, they accordingly exist in enantiomeric forms. If desired, the individual enantiomers may be isolated in pure form by conventional means. For example, a racemic mixture may be resolved into its component enantiomers by preparative chiral HPLC, or by treatment with an optically pure amine to form diastereomeric salt pairs, separable by fractional crystallisation, from which the optically pure acids may be regenerated. Similarly, a racemic acid may be reacted with an optically pure alcohol or amine to form pairs of diastereomeric esters or amides which may be separated by chromatography or fractional crystallisation and hydrolysed to yield enantiomerically-pure acids. These resolution techniques may equally well be practised on the synthetic precursors of the compounds of Formula I, and the resulting optically-pure intermediates used to prepare compounds of Formula I in optically-pure form.

A preferred synthetic route, capable of providing single enantiomers such as those of formula IIIA, IIIB, IIIC or IIID in which Y is CO₂H and both R² groups are H involves condensation of a hydrazine (5a) or 5(b) with an acrylic acid derivative (10) to form (11a) or (11b) respectively, followed by asymmetric hydrogenation of the exocyclic double bond: 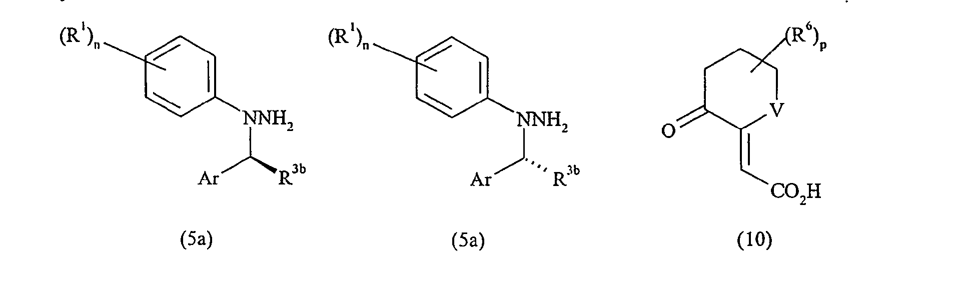 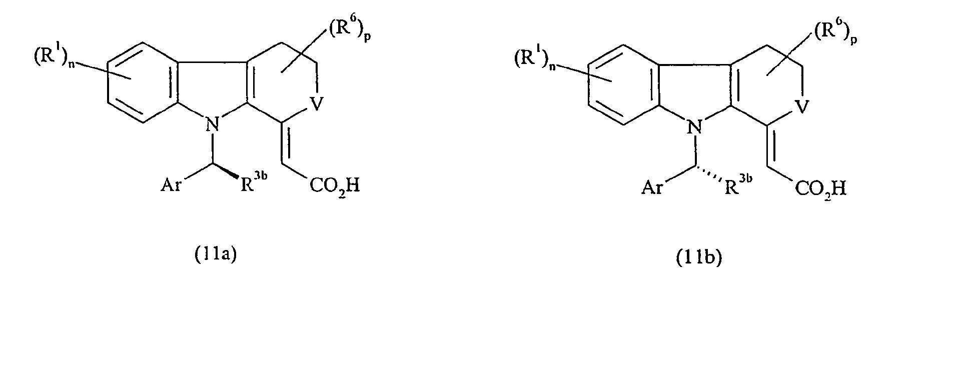 where V, Ar, n, p, R¹ and R⁶ have the same meanings as before, and R^{3b} is R³ that is other than H. The condensation is preferably carried out using excess of (10) in refluxing isopropanol in the presence of toluenesulphonic acid and molecular sieves. The hydrogenation is preferably carried out in a lower alkanol such as methanol over a chiral Ru(BINAP)Cl₂ catalyst, where BINAP is bis(diphenylphosphino)-1,1'-binaphthyl. Hydrogenation of (11a) over Ru(S-BINAP)Cl₂ is preferred.

Chiral hydrazines (5a) and (5b) may be obtained by alkylation of hydrazines (2) with chiral bromides (12a) and (12b) respectively, which in turn are obtainable, respectively, by treatment of chiral alcohols (13a) and (13b) with carbon tetrabromide and triphenylphosphine: 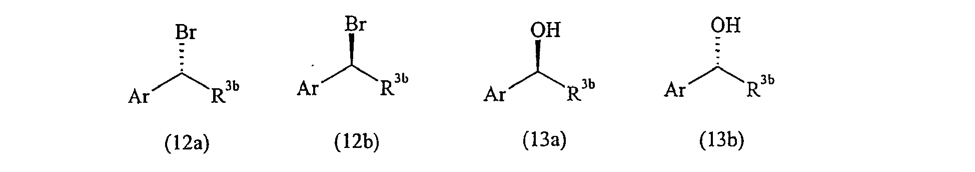 The alkylation may be carried out in THF in the presence of strong base such as sodium hexamethyldisilazide. The bromination is typically carried out in dichloromethane solution.

Chiral alcohols (13a) and (13b) are obtainable by asymmetric reduction of ketones Ar-CO-R^{3b}, where Ar and R^{3b} have the same meanings as before. Any suitable chiral reducing agent may be used, but in a preferred method reduction is effected using borane in the presence of a chiral oxazaborolidine (OAB) catalyst (see Corey, Angew, Chem. Int. Ed. Engl., 37 (1998), 1986). The reaction may be carried out in a dichloromethane/toluene mixture at -30°C. Use of (R)-OAB provides alcohol (13a) and (ultimately) hydrazine (5a). Use of (S)-OAB provides alcohol (13b) and (ultimately) hydrazine (5b).

The compounds of Formula I are typically used in the form of pharmaceutical compositions comprising one or more compounds of Formula I and a pharmaceutically acceptable carrier. Preferably these compositions are in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration by inhalation or insufflation. The principal active ingredient typically is mixed with a pharmaceutical carrier, e.g. conventional tableting ingredients such as corn starch, lactose, sucrose, sorbitol, talc, stearic acid, magnesium stearate and dicalcium phosphate, or gums, dispersing agents, suspending agents or surfactants such as sorbitan monooleate and polyethylene glycol, and other pharmaceutical diluents, e.g. water, to form a homogeneous preformulation composition containing a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This preformulation composition is then subdivided into unit dosage forms of the type described above containing from 0.1 to about 500 mg of the active ingredient of the present invention. Typical unit dosage forms contain from 1 to 100 mg, for example 1, 2, 5, 10, 25, 50 or 100 mg, of the active ingredient. Tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the compositions useful in the present invention may be incorporated for administration orally or by injection include aqueous solutions, liquid- or gel-filled capsules, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, poly(ethylene glycol), poly(vinylpyrrolidone) or gelatin.

For treating or preventing Alzheimer's disease, a suitable dosage level is about 0.01 to 250 mg/kg per day, preferably about 0.01 to 100 mg/kg per day, and more preferably about 0.05 to 50 mg/kg of body weight per day, of the active compound. The compounds may be administered on a regimen of 1 to 4 times per day. In some cases, however, a dosage outside these limits may be used.

The compounds of Formula I optionally may be administered in combination with one or more additional compounds known to be useful in the treatment or prevention of AD or the symptoms thereof. Such additional compounds thus include cognition-enhancing drugs such as acetylcholinesterase inhibitors (e.g. donepezil and galanthamine), NMDA antagonists (e.g. memantine) or PDE4 inhibitors (e.g. Ariflo™ and the classes of compounds disclosed in WO 03/01S579, WO 01/46151, WO 02/074726 and WO 02/098878). Such additional compounds also include cholesterol-lowering drugs such as the statins, e.g. simvastatin. Such additional compounds similarly include compounds known to modify the production or processing of Aβ in the brain ("amyloid modifiers"), such as compounds which inhibit the secretion of Aβ (including γ-secretase inhibitors, β-secretase inhibitors, and GSK-3α inhibitors), compounds which inhibit the aggregation of Aβ, and antibodies which selectively bind to Aβ.

In this embodiment of the invention, the amyloid modifier may be a compound which inhibits the secretion of Aβ, for example an inhibitor of of γ-secretase (such as those disclosed in WO 01/53255, WO 01/66564, WO 01/70677, WO 01/90084, WO 01/77144, WO 02/30912, WO 02/36555, WO 02/081435, WO 02/081433, WO 03/018543, WO 03/013506, WO 03/013527, WO 03/014075, WO 03/093252, WO 03/093264, WO 03/093251, WO 03/093253, WO 2004/039800 and WO 2004/039370), or a β-secretase inhibitor (such as those disclosed in WO 03/037325, WO 03/030886, WO 03/006013, WO 03/006021, WO 03/006423, WO 03/006453, WO 02/002122, WO 01/70672, WO 02/02505, WO 02/02506, WO 02/02512, WO 02/02520, WO 02/098849 and WO 02/100820), or any other compound which inhibits the formation or release of Aβ, including those disclosed in WO 98/28268, WO 02/47671, WO 99/67221, WO 01/34639, WO 01/34571, WO 00/07995, WO 00/38618, WO 01/92235, WO 01/77086, WO 01/74784, WO 01/74796, WO 01/74783, WO 01/60826, WO 01/19797, WO 01/27108, WO 01/27091, WO 00/50391, WO 02/057252, US 2002/0025955 and US2002/0022621, and also including GSK-3 inhibitors, particularly GSK-3α inhibitors, such as lithium, as disclosed in Phiel et al, Nature, 423 (2003), 435-9.

Within this embodiment, the amyloid modifier is advantageously a γ-secretase inhibitor, preferred examples of which include a compound of formula XI: 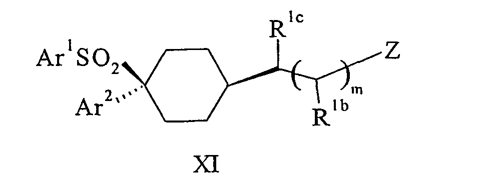 wherein m, R^{1b}, R^{1c}, Z, Ar¹ and Ar² are as defined in WO 03/018543;
or a pharmaceutically acceptable salt thereof.

Such compounds may be prepared as described in WO 03/018543. Preferred examples include those defined by formula XIa: 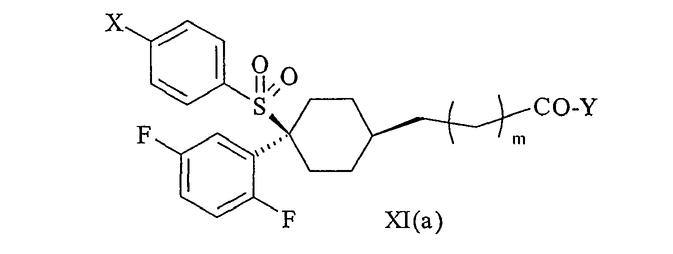 and the pharmaceutically acceptable salts thereof, wherein m is 0 or 1, X is Cl or CF₃, and Y is OH, OC₁₋₆alkyl, NH₂ or NHC₁₋₆alkyl. Particular examples include those in which m is 1 and Y is OH (or the sodium salts thereof), and those in which m is 0 and Y is NH₂ or NHC₁₋₆alkyl.

Another preferred class of γ-secretase inhibitors for use in this embodiment of the invention is that defined by formula XII: 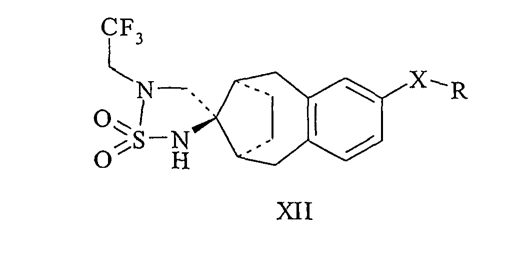 wherein X and R are as defined in WO 03/093252;
or a pharmaceutically acceptable salt thereof.

X is very aptly 5-substituted-thiazol-2-yl, 5-substituted-4-methylthiazol-2-yl, 5-substituted-1-methylpyrazol-3-yl, 1-substituted-imidazol-4-yl or 1-substituted-1,2,4-triazol-3-yl. Preferably, R represents optionally-substituted phenyl or heteroaryl such as phenyl, monohalophenyl, dihalophenyl, trihalophenyl, cyanophenyl, methylphenyl, methoxyphenyl, trifluoromethylphenyl, trifluoromethoxyphenyl, pyridyl, monohalopyridyl and trifluoromethylpyridyl, wherein "halo" refers to fluoro or chloro. Particularly preferred identities of R-X- include 5-(4-fluorophenyl)-1-methylpyrazol-3-yl, 5-(4-chlorophenyl)-1-methylpyrazol-3-yl and 1-(4-fluorophenyl)imidazol-4-yl. Such compounds may be prepared by methods disclosed in WO 03/093252.

Alternatively, the amyloid modifier may be a compound which inhibits the aggregation of Aβ. Suitable examples include chelating agents such as clioquinol (Gouras and Beal, Neuron, 30 (2001), 641-2) and the compounds disclosed in WO 99/16741, in particular that known as DP-109 (Kalendarev et al, J. Pharm. Biomed. Anal., 24 (2001), 967-75). Other inhibitors of Aβ aggregation suitable for use in the invention include the compounds disclosed in WO 96/28471, WO 98/08868 and WO 00/052048, including the compound known as Apan™ (Praecis); WO 00/064420, WO 03/017994, WO 99/59571 and the compcund known as Alzhemed™ (Neurochem); WO 00/149281 and the compositions known as PTI-777 and PTI-00703 (ProteoTech); WO 96/39834, WO 01/83425, WO 01/55093, WO 00/76988, WO 00/76987, WO 00/76969, WO 00/76489, WO 97/26919, WO 97/16194, and WO 97/16191.

Alternatively, the amyloid modifier may be an antibody which binds selectively to Aβ. Said antibody may be polyclonal or monoclonal, but is preferably monoclonal, and is preferably human or humanized. Preferably, the antibody is capable of sequestering soluble Aβ from biological fluids, as described in WO 03/016466, WO 03/016467, WO 03/015691 and WO 01/62801. Suitable antibodies include humanized antibody 266 (described in WO 01/62801) and the modified version thereof described in WO 03/016466.

As used herein, the expression "in combination with" requires that therapeutically effective amounts of both the compound of Formula I and the additional compound are administered to the subject, but places no restriction on the manner in which this is achieved. Thus, the two species may be combined in a single dosage form for simultaneous administration to the subject, or may be provided in separate dosage forms for simultaneous or sequential administration to the subject. Sequential administration may be close in time or remote in time, e.g. one species administered in the morning and the other in the evening. The separate species may be administered at the same frequency or at different frequencies, e.g. one species once a day and the other two or more times a day. The separate species may be administered by the same route or by different routes, e.g. one species orally and the other parenterally, although oral administration of both species is preferred, where possible. When the additional compound is an antibody, it will typically be administered parenterally and separately from the compound of Formula I.

In a further aspect, the invention provides the combination of a compound of formula I or a pharmaceutically acceptable salt thereof and a compound of formula XI(a) or a pharmaceutically acceptable salt thereof for use in treatment or prevention of a disease associated with deposition of β-amyloid in the brain. Said use may involve the simultaneous or separate administration of the respective compounds to a patient in need of such treatment or prevention..

In a further aspect, the invention provides a pharmaceutical composition comprising, in a pharmaceutically acceptable carrier, a compound of formula I or a pharmaceutically acceptable salt thereof and a compound of formula XI(a) or a pharmaceutically acceptable salt thereof. Preferably, the pharmaceutical composition is in a unit dose form suitable for oral administration, such as a tablet or a capsule.

### EXAMPLES

The ability of the compounds of Formula I to selectively inhibit production of Aβ(1-42) was determined using the following assay:

### Cell-based γ-Secretase Assay

Human SH-SY5Y neuroblastoma cells overexpressing the direct γ-secretase substrate SPA4CT were induced with sodium butyrate (10 mM) for 4 hours prior to plating. Cells were plated at 35,000 cells/well/100 µl in 96-well plates in phenol red-free MEM/10% FBS, 50 mM HEPES, 1% Glutamine and incubated for 2 hrs at 37 °C, 5% CO₂.

Compounds for testing were diluted into Me₂SO to give a ten point dose-response curve. Typically 10 µl of these diluted compounds in Me₂SO were further diluted into 182 µl dilution buffer (phenol red-free MEM/10% FBS, 50 mM HEPES, 1% Glutamine) and 10 µl of each dilution was added to the cells in 96-well plates (yielding a final Me₂SO concentration of 0.5%). Appropriate vehicle and inhibitor controls were used to determine the window of the assay.

After incubation overnight at 37 °C, 5%CO_{2,} 10 µl and 50 µl media were transferred into a fresh Costar round-bottom 96-well plate for detection of Aβ(40) and Aβ(42) peptides, respectively. 40 µl Origen buffer (PBS, 2% BSA, 0.2% Tween-20) was added to the Aβ(40) wells followed by the addition of 25 µl the respective antibody premixes to the wells:
Aβ(40) premix: 1 µg/ml ruthenylated G2-10 antibody, 4 µg/ml biotinylated 4G8 antibody diluted in Origen buffer
Aβ(42) premix: 0.5 µg/ml ruthenylated G2-11 antibody, 4 µg/ml biotinylated 4G8 antibody diluted in Origen buffer

### (Biotinylated 4G8 antibody supplied by Signet Pathology Ltd; G2-10 and G2-11 antibodies supplied by Chemicon)

After overnight incubation of the assay plates on a shaker at 4 °C, the Origen M8 Analyser (Igen Inc.) was calibrated according to the manufacturer's instructions. 25 µl of streptavidin magnetic bead (Dynal) premix (400 µg/ml streptavidin beads/ml in Origen buffer) was added to the assay plates and incubated on a shaker for 15 minutes. 150 µl Origen buffer was added to each well and the plates were read on the Origen M8 Analyser according to the manufacturer's instructions.

Cell viability was measured in the corresponding cells after removal of the media for the Aβ assays by a colorimetric cell proliferation assay (CellTiter 96^{™} AQ assay, Promega) utilizing the bioreduction of MTS (Owen's reagent) to formazan according to the manufacturer's instructions. Briefly, 5 µl of 10x MTS/PES was added to the remaining 50 µl of media before returning to the incubator. The optical density was read at 495 nm after ~4 hours.

LD₅₀ and IC₅₀ values for inhibition of Aβ(40) and Aβ(42) were calculated by nonlinear regression fit analysis using the appropriate software (eg. Excel fit). The total signal and the background were defined by the corresponding Me₂SO and inhibitor controls.

The compounds listed in Tables 1-3 above all gave IC₅₀ values for Aβ(1-42) inhibition that were at least 2-fold lower than the corresponding IC₅₀ values for Aβ(1-40) inhibition, typically at least 5-fold lower, and in the preferred cases at least 50-fold lower.

### EXAMPLE 1

### {6-Isopropyl-9-[1-(4-trifluoromethyl-phenyl)-ethyl]-2,3,4,9-tetrahydro-1H-carbazol-1-yl}-acetic acid

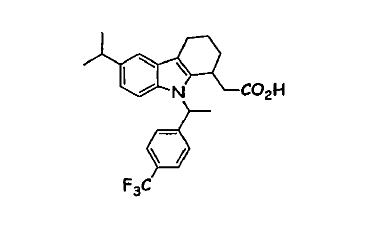

### Step 1

To a stirred solution of 4-isopropylphenylhydrazine hydrochloride (8.35g, 45mmol.) in ethanol (300ml) was added ethyl 2-cyclohexanoneacetate (8.23g, 45mmol.) and the mixture heated to reflux for 16 hours. Upon cooling, the solvent was evaporated and the residue taken up in ethyl acetate (200ml) and washed with 1N HCl (200ml). The aqueous was extracted with further ethyl acetate (200ml) and the combined organics washed with brine (100ml), dried (MgSO₄) and evaporated to dryness. The residue was purified by column chromatography eluting with ether: hexane (1:3) to afford the desired tetrahydrocarbazole (5.1g). ¹H NMR (CDCl₃) 8.60 (1H, br s), 7.29 (1H, d, J=1.0Hz), 7.21 (1H, d, J=8.5Hz), 7.01 (1H, dd, J=8.5, 1.0Hz), 4.20 (2H, q, J=7.0Hz), 3.34 (1H, m), 2.99 (1H, septet, J=7.0Hz), 2.71-2.54 (4H, m), 2.05 (1H, m), 1.95-1.75 (2H, m), 1.69-1.57 (1H, m), 1.30 (6H, d, J=7.0Hz), 1.28 (3H, t, J=7.0Hz). m/z =300 [MH]⁺

### Step 2

To a degassed solution of the tetrahydrocarbazole from the foregoing step (448mg, 1.50mmol) in DMF (10ml) was added potassium tert-butoxide (201mg, 1.8mmol). The resulting red/brown solution was stirred at ambient temperature for 10 minutes before a solution of 1-(1-bromoethyl)-4-trifluoromethylbenzene (417mg, 1.65mmol.) in DMF (2ml) was added. After stirring an additional 2.75 hours, the colour had faded to pale yellow and the reaction was quenched by the addition of 2N HCl (50ml). The mixture was extracted with ethyl acetate (2x100ml) and the combined organics washed with further 2N HCl (100ml), water (100ml) and brine (100ml), dried (MgSO₄) and evaporated to dryness. The residue was purified by column chromatography eluting with ether: hexane (1:6) to afford the desired N-benzylated tetrahydrocarbazole (376mg) as a 3:2 mixture of diastereomers (designated isomer A and isomer B respectively). ¹H NMR (CDCl₃) 7.56 (2H, d, J=8.5Hz, isomer B), 7.50 (2H, d, J=8.5Hz, isomer A), 7.33 (3H [isomer B] + 1H [isomer A], m), 7.19 (2H, d, J=8.5Hz, isomer A), 6.87-6-81 (2H [isomer A] + 1H [isomer B], m), 6.63 (1H, d, J=8.5Hz, isomer B), 5.63 (1H, br q, J=7.0Hz, isomers A+B), 4.24-4.02 (2H, m, isomers A+B), 3.49 (1H, br d, J=10Hz, isomers A+B), 3.00-2.83 (2H, m, isomers A+B), 2.73-2.37 (4H, m, isomers A+B), 2.05 (3H, d, J=7.0Hz, isomer A), 2.01-1.80 (3H, m, isomers A+B), 1.87 (3H, d, J=7.0Hz, isomer B), 1.32-1.18 (9H, m, isomers A+B). m/z =472 [MH]⁺.

### Step 3

To a solution of the ester from the foregoing step (376mg, 0.80mmol) in THF (20ml) was added a solution of lithium hydroxide (200mg) in water (10ml) and the mixture stirred vigorously at 60°C for 4 hours. Upon cooling, the mixture was partitioned between ethyl acetate (50ml) and 2N HCl (50ml). The aqueous was extracted with further ethyl acetate (50ml) and the combined organics washed with water (50ml) and brine (50ml), dried (MgSO₄) and evaporated to dryness. The residue was purified by column chromatography eluting with acetic acid: ethyl acetate: hexane (0:33:100 to 1:33:100) to afford the desired product (340mg) as a 3:2 mixture of diastereomers (designated isomer A and isomer B respectively). ¹H NMR (CDCl₃) 11.5-9.5 (1H, v br s, isomer A+B), 7.57 (2H, d, J=8.5Hz, isomer B), 7.51 (2H, d, J=8.5Hz, isomer A), 7.33 (3H [isomer B] + 1H [isomer A], m), 7.18 (2H, d, J=8.5Hz, isomer A), 6.86-6-82 (2H [isomer A] + 1H [isomer B], m), 6.64 (1H, d, J=B.5Hz, isomer B), 5.60 (1H, br q, J=7.0Hz, isomers A+B), 3.49 (1H, m, isomers A+B), 3.01-2.81 (2H, m, isomers A+B), 2.75-2.43 (4H, m, isomers A+B), 2.05 (3H, d, J=7.0Hz, isomer A), 2.01-1.80 (3H, m, isomers A+B), 1.88 (3H, d, J=7.0Hz, isomer B), 1.26 (6H, m, isomers A+B). m/z =444 [MH]⁺.

This racemic mixture of diastereomers could be efficiently separated into the four individual stereoisomers by supercritical fluid chromatography using a Berger Instruments Minigram SFC. Column: Chiralcel OJ-H 250x10mm (5µ) [Chiral Technologies] at oven temperature 35°C, eluent CO₂ + 8% [MeOH+0.1% diethylamine] modifier run at 10ml/min with CO₂ outlet pressure 100 bar; detection at 220nm.

Isomer B, ent. 1 at 5.68 min.; Isomer B, ent. 2 at 6.21 min.; Isomer A, ent. 1 at 7.19 min.; Isomer A, ent. 2 at 9.49 min.

### Preparation of 1-(1-bromoethyl)-4-trifluoromethylbenzene.

To a stirred solution of 1-(4-trifluoromethylphenyl)-ethanol (4.2g, 22mmol.) in dichloromethane (60ml) under nitrogen was added dropwise phosphorus tribromide (2.3ml, 24mmol.). The reaction was stirred 1 hour at ambient temperature then quenched by the addition of water (20ml). The organic layer was washed with further water (30ml), a saturated solution of sodium bicarbonate (30ml) and brine (30ml) then dried (MgSO₄) and evaporated to afford the product (3.6g). ¹H NMR (CDCl₃) 7.60 (2H, d, J=8.5Hz 7.54 (2H, d, J=8.5Hz), 5.19 (1H, q, J=7.0Hz), 2.04 (3H, d, J=7.0Hz).

### EXAMPLE 2

### {4-(4-fluorophenyl)-6-isopropyl-9-[(4-trifluoromethylphenyl)methyl]-1,2,3,4-tetrahydrocarbazole-l-yl} acetic acid

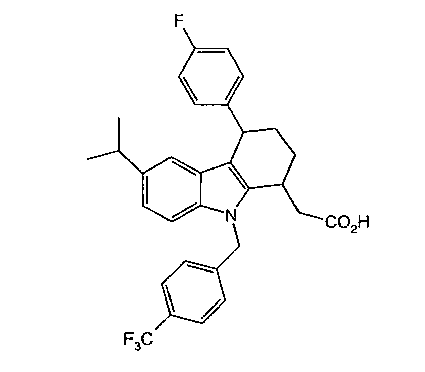

### Step 1

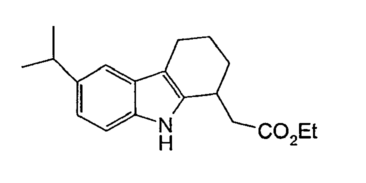

Available by the procedure described in EP0234708, Example 34 Step 1, using 4-isopropylphenylhydrazine hydrochloride.

### Step2

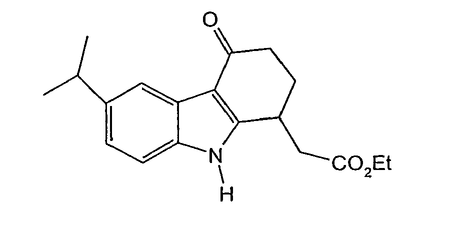

The product of Step 1 (5.0 g, 16.7 mmol) in degassed THF (170 ml) and water (15 ml), under nitrogen, was cooled to 0°C and DDQ (9.3 g, 41.4 mmol) in degassed THF (60 ml) was added dropwise over 15 min. After 3 h at 0°C the mixture was concentrated, the residue taken up in ethyl acetate (200 ml), then washed with saturated aqueous sodium hydrogencarbonate (5x50 ml), water, brine, dried (MgSO₄ and evaporated to give a tan solid (4.8 g).
m/z=314 [MH]⁺

### Step 3

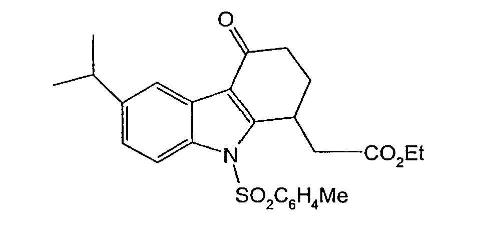

The product of Step 2 (1,6 g, 7.5 mmol) in dry DMF (25 ml) was cooled to 0°C under nitrogen. Sodium hydride (60% dispersion in mineral oil, 450 mg, 11.3 mmol) was added portionwise over 15 min. After stirring at 0°C for 20 min, tosyl chloride (2.1 g, 11.3 mmol) in dry toluene (25 ml) was added dropwise. The reaction was stirred at 0°C for 1 h and then allowed to warm to room temperature and stirred for a further 1 h. The reaction was quenched with saturated aqueous ammonium chloride (5 ml), diluted with water (500 ml) and extracted with ethyl acetate (3x100 ml). The organic extracts were washed with water (2x 50 ml), brine, dried (MgSO₄) and evaporated. The crude product was purified by flash chromatography eluting with 9:1 *ⁱ*hexane/ethyl acetate to 4:1 *ⁱ*hexane/ethyl acetate to afford a light brown foam (1.9 g). ¹H NMR (CDCl₃) 8.10 (2H, dd, J= 8.4, 1.8Hz), 7.73 (2H, d, J = 8.4 Hz), 7.32 (3H, m), 4.23 (2H, q, J = 7.0Hz), 3.05-2.98 (2H, m), 2.75-2.65 (2H, m), 2.53-2.48 (2H, m), 2.37 (3H, s), 2.30-2.20 (2H, m), 1.30 (6H, d, J=7.1Hz), 1.27 (3H, t, J = 7.0Hz).
m/z = 468 [MH]⁺

### Step 4

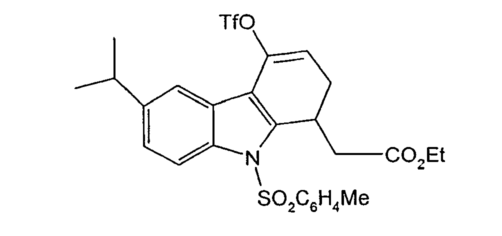

To the product of Step 3 (1.7 g, 4.6 mmol) in dry THF (20 ml) under nitrogen and cooled to -78°C was added lithium hexamethyldisilazide (1 M solution in THF, 7 ml, 7 mmol) dropwise over 15 min. After stirring at -78°C for 1 h, N-phenylbis(trifluoromethanesulfonimide) (2.5 g, 6.9 mmol) in dry THF (20 ml) was added over 10 min. The reaction was stirred at -78°C for 1 h and then at 0°C for 1 h before being quenched with saturated aqueous ammonium chloride (5 ml), diluted with water (300 ml) and extracted with ether (3x100 ml). The organic extracts were washed with water, brine, dried (MgSO₄) and evaporated. The crude product was purified by flash chromatography eluting with 5:1 *ⁱ*hexane/ether to afford a colourless foam (1.4 g).
¹H NMR (CDCl₃) 8.11 (1H, d, J = 8.8Hz), 7.75 (2H, d, J = 8.4Hz), 7.58 (1H, d, J = 1.4Hz), 7.30-7.23 (3H, m), 5.68 (1H, dd, J = 2.8, 6.7Hz), 4.17 (2H, q, J = 7.0Hz), 4.02 (1H, m), 3.00 (1H, septet, J = 7.1 Hz), 2.68-2.52 (4H, m), 2.37 (3H, s), 1.30 (6H, d, J = 7.0Hz), 1.27 (3H, t, J = 7.0Hz).

### Step 5

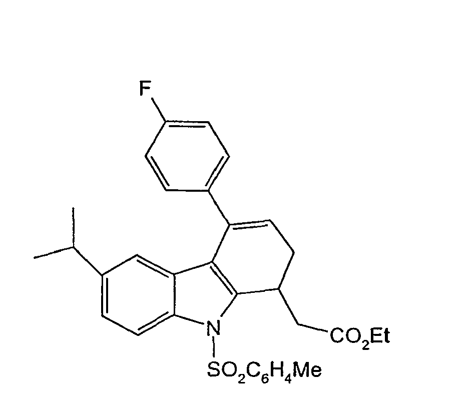

To the product of Step 4 (500 mg, 0.83 mmol) in dioxane (25 ml) was added 4-fluorobenzeneboronic acid (128 mg, 0.91 mmol), potassium phosphate (264 mg, 1.2 mmol) and Pd(PPh₃)₄ (50 mg, 5 mol%). The mixture was heated at 80°C for 3 h, allowed to cool, diluted with water (200 ml) and extracted with ethyl acetate (3x50 ml). The organic extracts were washed with brine, dried (MgSO₄) and evaporated. The product was purified by flash chromatography eluting with 9:1 *ⁱ*hexane/ethyl acetate to 5:1 *ⁱ*hexane/ethyl acetate to afford a colourless solid (395 mg). m/z = 546 [MH]⁺
¹H NMR (CDCl₃) 8.12 (1 H, d, J = 8.8Hz), 7.77 (2H, d, J = 8.4Hz), 7.43-7.22 (5H, m), 7.12-7.02 (2H, m), 6.43 (1H, d, J = 1.8Hz), 5.72 (1H, dd, J = 2.8, 6.7Hz), 4.19 (2 H, q, J = 7.1Hz), 4.13-4.06 (1H, m), 2.78-2.61 (4H, m), 2.54-2.47 (1 H, m), 2.35 (3 H, s), 1.27 (6 H, d, J=7.0Hz), 1.26 (3H, t, J = 7.0 Hz).

### Step 6

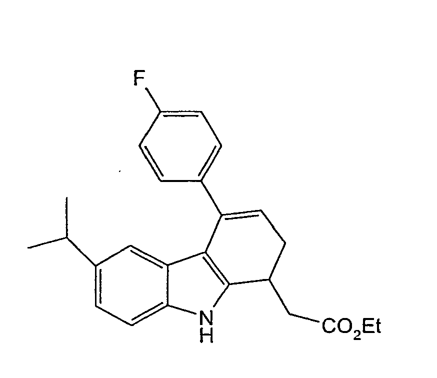

The product of Step 5 (390 mg, 0.78 mmol) in a 1:1 mix of THF/methanol (10 ml) was treated with sodium dihydrogen phosphate (365 mg, 2.3 mmol) and sodium-mercury amalgam (5% sodium, 700 mg, excess). After stirring at room temperature for 2 h the reaction was decanted, diluted with water (100 ml) and extracted with ethyl acetate (3x50 ml). The organic extracts were washed with brine, dried (MgSO₄) and evaporated to give a yellow oil (290 mg).
m/z = 378 [MH]⁺

### Step 7

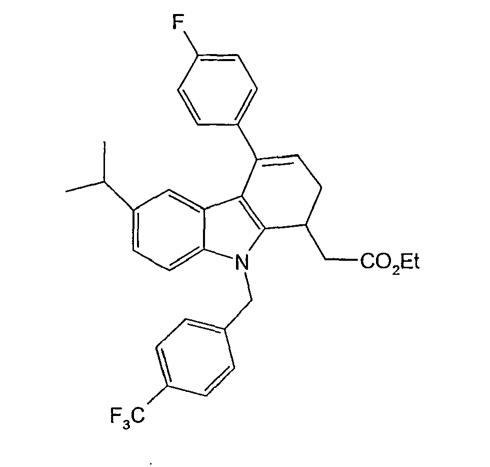

The product of Step 6 (200 mg, 0.5 mmol) in dry DMF (7 ml) was cooled to 0°C under nitrogen, sodium hydride (60% dispersion in mineral oil, 25 mg, 0.55 mmol) was added, the reaction was stirred at 0°C for 20 min, and then 4-(trifluoromethyl)benzyl bromide (350 mg, 0.6 mmol) was added. The reaction was allowed to warm to room temperature, stirred for 18 h, then diluted with water (100 ml) and extracted with ethyl acetate (3x50 ml). The organic extracts were washed with brine, dried (MgSO₄) and evaporated. The product was purified by flash chromatography eluting with 9:1 *ⁱ*hexane/ethyl acetate to 4:1 *ⁱ*hexane/ethyl acetate to afford a light green oil (180 mg).
¹H NMR (CDCl₃) 7.54 (2H, d, J = 8.4Hz), 7.48-7.43 (2H, m), 7.15-7.07 (5H, m), 7.00-6.97 (1H, m), 6.72 (1H, s), 5.58 (1 H, dd, J = 2.8, 6.7Hz), 5.56 (1H, d, J= 16Hz), 5.43 (1H, d, J = 16Hz,), 3.61 (3 H, s), 3.41-3.34 (1H, m), 2.84-2.64 (3H, m), 2.54-2.47 (1H, m), 2.32 (1H, dd, J = 5.1, 16.0Hz), 1.10 (6H, d, J = 7.0Hz).
m/z =536 [MH]⁺

### Step 8

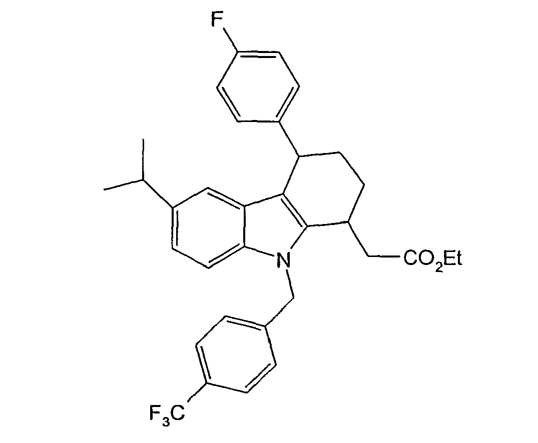

To the product of Step 7 (175 mg, 0.33 mmol) in ethyl acetate (25 ml) was added 10% palladium on carbon (25 mg). The mixture was shaken under an atmosphere of hydrogen at 50 psi for 36 h. The catalyst was removed by filtration, the filtrate evaporated, and the residue purified by flash chromatography eluting with 9:1 *ⁱ*hexane/ethyl acetate to 4:1 *ⁱ*hexane/ethyl acetate to give a colourless oil (155 mg).
m/z =538 [NM]⁺

### Step 9

To the product from Step 8 (140 mg, 0.26 mmol) in THF (4 ml) was added lithium hydroxide (35 mg, 1.25 mmol) in water (1 ml). The reaction was stirred for 18 h and then diluted with water (100 ml), acidified with 2 M hydrochloric acid and extracted with ethyl acetate (3x50 ml). The organic extracts were washed with brine, dried (MgSO₄) and evaporated. The crude product was purified by flash chromatography eluting with 1:1 *ⁱ*hexane/ethyl acetate to ethyl acetate to give the title compound as a colourless solid (85 mg).
¹H NMR (CDCl₃) 7.54 (2H, d, J = 8.0Hz,), 7.20-7.24 (2H, m), 7.10 (2H, d, J = 8.0Hz), 6.90-7.02 (4H, m), 6.52 (1 H, d, J = 1.4Hz), 5.39 (1H, d, J = 17 Hz), 5.32 (1H, d, J = 17Hz,), 4.09-4.17 (1 H, m), 3.42 (1 H, t, J = 1.6Hz), 2.63-2.79 (2 H, m), 2.52-2.57 (1 H, m), 2.29-2.37 (1 H, m), 2.13-2.21 (2 H, m), 1.72-2.00 (2H, m), 1.05-1.29 (6 H, m).
m/z =524 [MH]⁺.

Following the same procedure, using the appropriate boronic acid in Step 5 and the appropriate benzyl halide in Step 7, there was prepared:

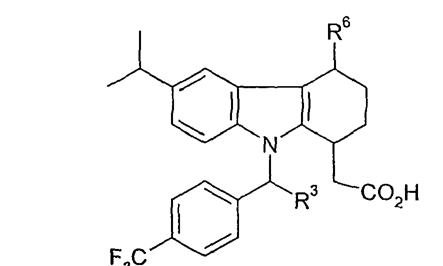

| **Example** | **R³** | **R⁶** | **mass spec [MH]⁺** |
|---|---|---|---|
| 2a | n-propyl | 4-F-Ph | 566 |
| 2b | H | 2,5-di-Me-Ph | 534 |
| 2c | H | Ph | 506 |
| 2d | H | 4-MeO-Ph | 536 |
| 2e | H | 4-F-3-Me-Ph | 538 |
| 2f | H | 4-Cl-Ph | 541 |
| 2g | H | 3-F-Ph | - |
| 2h | H | 3,4-di-F-Ph | - |
| 2i | H | 3-pyridyl | 507 |
| 2j | H | isopropyl | 472 |

### EXAMPLE 3

Following the procedure of Example 1, using the appropriate phenylhydrazine in Step 1 and the appropriate 1-(1-bromoalkyl)-4-trifluoromethylbenzene in Step 2, the following were prepared:

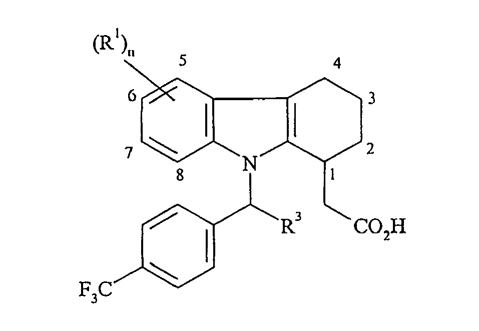

| **Example** | **(R¹)ₙ** | **R³** | **mass spec*** |
|---|---|---|---|
| 3a | 6-Br | n-propyl | 508/510 |
| 3b | 7-Br | n-propyl | 508/510 |
| 3c | 5-Br | n-propyl | 508/510 |
| 3d | 6-^{t}butyl | n-propyl | 486 |
| 3e | 6-OMe | n-propyl | 460 |
| 3f | 6-isopropoxy | n-propyl | - |
| 3g | 6-cyclohexyl | n-propyl | 512 |
| 3h | 6-isopropyl | ethyl | 458 |
| 3i | 6-isopropyl | n-propyl | 472 |
| 3j | 6,8-dichloro | ethyl | 482, 484 (M-H) |
| 3k | 6,8-dichloro | n-propyl | 498, 500 |
| 31 | 5,7-dichloro | n-propyl | 498,500 |
| 3m | 6-isopropyl | cyclohexyl | 512 |
| 3n | 6-isopropyl | isopropyl | 472 |
| 3o | 6-isopropyl | 3-methylbutyl | 500 |
| 3p | 6-isopropyl | CF₃CH₂CH₂ | 526 |
| 3q | 6-isopropyl | n-butyl | 486 |
| 3r | H | n-propyl | 428 (M-H) |
| 3s | 8-Cl-6-isopropyl | n-propyl | 505 (M-H) |
| 3t | 5-bromo-8-fluoro | H | 484 (M-H) |
| 3u | 5-bromo-8-chloro | H | 478 (M-H) |
| 3v | 5,7-dimethyl | H | 416 |
| 3w | 8-bromo | H | 464 (M-H) |
| 3x | 5-bromo | H | 464 (M-H) |
| 3y | 7-bromo | H | 464 (M-H) |
| 3z | 5-(3,4-di-Cl-Ph)- | H | 532 (M-H) |
| 3aa | 6-chloro | n-propyl | 464 |
| 3bb | 6-isopropyl | 2-methylpropyl | 486 |
| 3cc | 8-chloro | n-propyl | 464 |
| 3dd | 5,8-dichloro | H | - |
| 3ee | 8-n-butyl-6-isopropyl | H | 486 |
| 3ff | 8-cyclopropyl-6-isopropyl | H | 470 |
| 3gg | 8-isopropyl | H | 430 |
| 3hh | 5,7-dichloro | H | 457 |
| 3ii | 6,8-difluoro | n-propyl | 466 |
| 3jj | 8-ethyl | H | 416 |
| 3kk | 8-chloro | H | 421 |
| 3ll | 8-(3,4-di-Cl-Ph)- | H | 532 |
| 3mm | 6-isopropyl | allyl | 488 |

| | | | |
|---|---|---|---|
| * [MH]⁺ unless otherwise indicated | | | |

### EXAMPLE 4

### (6-Butyl-9-{1-[4-(trifluoromethyl)phenyl]propyl}-2,3,4,9-tetrahydro-1H-carbazol-1-yl)acetic acid

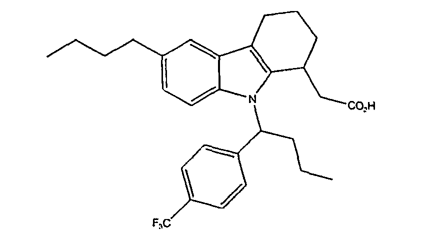

To the product from Example 3a, Step 2 (55 mg, 0.1 mmol) in toluene (2 ml) and water (0.1 ml) were added butylboronic acid (15 mg, 0.134 mmol), potassium phosphate (75 mg, 0.35 mmol), tricyclohexylphosphine (3 mg, 0.01 mmol) and palladium acetate (3 mg, 0.01 mmol). The mixture was degassed, placed under nitrogen and heated at 100°C for 5h. After cooling the reaction was diluted with EtOAc (50 ml), passed through Celite and the filtrate washed with water (20 ml), brine (20 ml), dried (MgSO₄), filtered and evaporated. The crude product was purified by flash chromatography eluting with *ⁱ*hexane to 20:1 *ⁱ*hexane/ether to give a colourless oil (25 mg) as a mix of diastereoisomers. To this ester (25 mg, 0.045 mmol) in THF (5 ml) under nitrogen was added a solution of lithium hydroxide (7 mg, 0.32 mmol) in water (1 ml). The reaction was stirred for 18 h and then diluted with water (30 ml), made acidic with hydrochloric acid (aqueous, 2 M) and extracted with EtOAc (3 x 20 ml). The organic extracts were washed with brine, dried (MgSO₄), filtered and evaporated. The crude product was purified by flash chromatography eluting with 4:1 *ⁱ*hexane/ethyl acetate to 1:1 *ⁱ*hexane/ethyl acetate to give (6-Butyl-9-{1-[4-(trifluoromethyl)phenyl]propyl}-2,3,4,9-tetrahydro-1H-carbazol-1- yl)acetic acid as a white solid (18 mg) as a 1:1 mix of diastereoisomers. ¹H NMR (CDCl₃) 7.54 (1H, J = 7.8 Hz), 7.48 (1H, J = 7.9 Hz), 7.24 (4H, m), 6.79 (1H, m), 5.41 (1H, t, J = 8.1 Hz), 3.50 (0.5 H, m, diastereomer A), 3.44 (0.5 H, m, diastereomer B), 2.82 (1H, m), 2.65 (3H, m), 2.52-2.45 (2H, m), 2.34-2.28 (1H, m), 2.0-1.76 (4H, m), 1.61 (2H, pent, J = 6.8 Hz), 1.41-1.18 (5H, m), 0.99 - 0.71 (6H, m). m/z = 486 [MH]⁺

Following the same procedure, using the appropriate alkylboronic acid and using the intermediate from Step 2 of Example 3a, 3b or 3c as appropriate, there was also prepared:

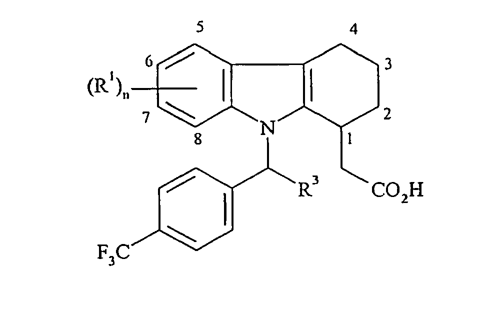

| **Example** | **(R¹)ₙ** | **R³** | **mass spec** |
|---|---|---|---|
| 4a | 6-cyclopropyl | n-propyl | 470 |
| 4b | 6-(2-methylpropyl) | n-propyl | 486 |
| 4c | 5-cyclopropyl | n-propyl | 468 (M-H) |
| 4d | 5-isopropyl | n-propyl | 472 |
| 4e | 7-cyclopropyl | n-propyl | 468 (M-H) |

### EXAMPLE 5

### (6-Cvano-9-{1-[4-(trifluoromethyl)phenyl]propyl}-2,3,4,9-tetrahydro-1H-carbazol-1-yl)acetic acid

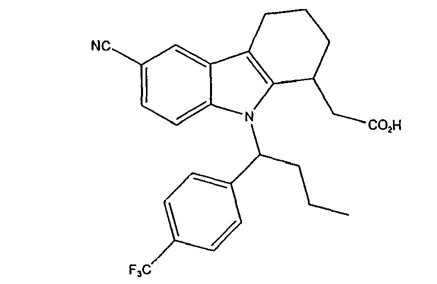

To the intermediate from Example 3a, Step 2 (85 mg, 0.16 mmol) in dry N-methyl-2-pyrrolidinone (5 ml) under nitrogen was added copper (I) cyanide (50 mg, 0.56 mmol). The mixture was heated at 180°C for 3 h. After cooling the reaction was diluted with water (50 ml) and extracted with diethyl ether (3 x 20 ml). The ether extracts were washed with aqueous ammonia solution (3 x 20 ml), water (20 ml) and brine (20 ml), dried (MgSO₄), filtered and evaporated. The crude product was purified by flash chromatography eluting with 9:1 *ⁱ*hexane/ether to 4:1 *ⁱ*hexane/ether to give a white solid (68 mg). The ester was hydrolysed as in Example 1, step 3 to give the title compound as a white solid (55 mg) as a ca. 1:1 mix of diastereoisomers. ¹H NMR (CDCl₃) 7.85 (1H, d, J =12.1 Hz), 7.59-7.51 (2H, m), 7.30-7.16 (3.5H, m), 6.86 (0.5H, d, J = 7.1 Hz), 5.48 (1H, t, J = 7.4 Hz), 3.59-3.49 (0.5H, m, diastereomerA), 3.47-3.39 (0.5H, m, diastereomer B), 2.91-2.75 (1H, m), 2.71-2.39 (3H, m), 2.01-1.95 (2H, m), 1.91-1.81 (2H, m), 1.33-1.18 (2H, m), 0.97 (2H, m), 0.85 (3H, t, J = 7.1 Hz). m/z = 455 [MH]⁺

### EXAMPLE 6

### (5-(morpholin-1-yl)-9-{1-[4-(trifluoromethyl)phenyl]propyl}-2,3,4,9-tetrahydro-1H-carbazol-1-yl)acetic acid

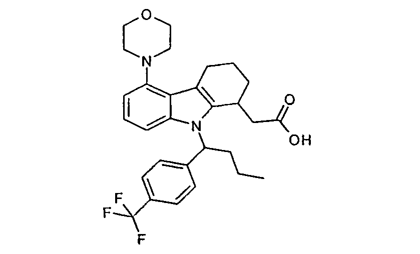

Potassium hydroxide (20 mg), cetyltrimethylammonium bromide (4mg) and bis(tri t-butylphosplino)palladium(0) (2mg) were mixed and the flask purged with nitrogen. The product from Example 3c, Step 2 (144mg) was dissolved in toluene (1.5ml) and added followed by morpholine (17µL) and water (5 µL) and the mixture heated at 90°C for 15hrs. The reaction was cooled to ambient temperature, water added and the mixture extracted with ethyl acetate (x3). The combined organics were washed with brine, evaporated in vacuo and purified by chromatography (Silica gel and 20:1 hexane:ethyl acetate) to give the desired ethyl (5-(morpholin-1-yl)-9-{1-[4-(trifluoromethyl)phenyl]propyl}-2,3,4,9-tetrahydro-1H -carbazol-1-yl)acetate as a mixture of diastereoisomers.(10mg). MH+ 543. This ester was hydrolysed using the procedure of example 1, step 3 to afford the desired (5-(morpholin-1-yl)-9-{1-[4-(trifluoromethyl)phenyl]propyl}-2,3,4,9-tetrahydro-1H -carbazol-1-yl)acetic acid (8mg) as a mixture of diastereoisomers ¹H NMR δ ppm (CDCl₃) 7.56 (1H, d, J= 8.4Hz), 7.52 (1H, d, J=8.2 Hz), 7.26 (0.5H, m), 7.18 (1H, d, J=7.85Hz), 7.14 (1H, d, J=8.25Hz), 7.01 (0.5H, t, J=7.95Hz), 6.85-6.97 (1.5H, m), 6.74 (0.5H, d, J=8.05), 5.40-5.55 (1H, m), 4.09 (4H, br s), 3.15-3.6 (5.5H, m), 2.78-2.95 (1H,m), 2.33-2.75 ( 4H, m), 1.70-2.10 (4H, m), 1.45-1.60 (0.5H, m), 1.15-1.40 (1.5H, m), 0.99 (1.5H, t, J=7.3Hz), 0.88 (1.5H, t, J=7.3Hz), 0.7-0.83 (0.5H, m). m/z 513 [MH+]

### EXAMPLE 7

### (5-(tert-Butyloxy)-9-{1-[4-(trifluoromethyl)phenyl]propyl}-2,3,4,9-tetrahydro-1H-carbazol-1-yl)acetic acid

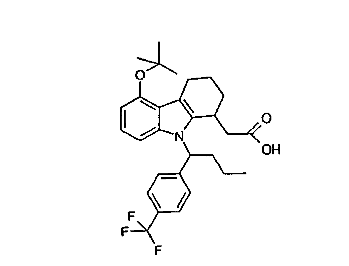

To a mixture of palladium acetate (2.6mg), 2-(di-t-butylphosphino)biphenyl (2mg) and sodium t-butoxide (27mg) in a nitrogen-purged flask was added toluene (0.5ml) and the product from Example 3c, Step 2 (99mg) as a solution in toluene (1ml). The reaction was heated at 100°C for 3hrs, cooled, water added and the mixture extracted with ethyl acetate (x2). The combined organic extracts were dried over MgSO₄, evaporated in vacuo and purified by chromatography using silica gel / 20:1 hexane: ethyl acetate to give the desired ethyl (5-(tert-butyloxy)-9- {1-[4-(trifluoromethyl)phenyl]propyl}-2,3,4,9-tetrahydro-1H -carbazol-1-yl)acetate (70mg). This ester was hydrolysed using the procedure of Example 1, Step 3 to afford the desired (5-(tert-butyloxy)-9-{1-[4-(trifluoromethyl)phenyl]propyl}-2,3,4,9-tetrahydro-1 H -carbazol-1-yl)acetic acid (50mg)
¹H NMR (CDCl₃) 7.45-7.60 (3H, m), 7.31 (1H, d, J=8.2Hz), 7.18 (1H, d, J=8.0), 6.95-7.1 (2H, m), 5.45 (1H, br s), 3.51 (0.5H, d, J=15Hz), 3.41 (0.5H, d, J=10Hz), 2.80-2.90 (1H, m), 2.25-2.75 (7H, m), 1.80-2.03 (4H, m), 1.15-1.60 (9H, m), 0.99 (1.5H, t, J=7.5Hz), 0.80-0.90 (1.5H, m)

### EXAMPLE 8

### (5-Cyano-9-{1-[4-(trifluoromethyl)phenyl]propyl}-2,3,4,9-tetrahydro-1H -carbazol-1-yl)acetic acid

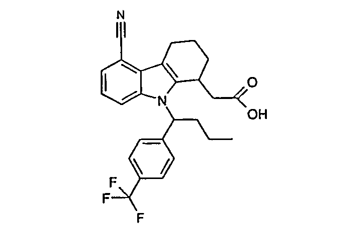

Prepared from the intermediate from Example 3c, Step 2 (100mg) using the method of Example 5 to give desired compound as a mixture of diastereoisomers (35mg) ¹H NMR δ ppm (CDCl₃) 7.57 (1H, d, J=8Hz), 7.53 (1H, d, J=15Hz), 7.35-7.45 (1H, m), 7.26 (1H, d, J=6Hz), 7.16 (1H, d, J=8Hz), 6.95-7.10 (2H, m), 5.5 (1H s), 3.4-3.6 (1H, m), 3.20-3.40 (1H, m), 2.80-3.0 (1H, m), 2.30-2.80 (4H, m), 2.17 (0.5H, s), 1.80-2.10 (4H, m), 1.45-1.60 (0.5H, m), 1.43 (0.5H, s), 1.15-1.35 (1H, m), 1.00 (1.5H, t, J=7.3Hz), 0.88 (2.0H, m). m/z = 453 [M-H]

### EXAMPLE 9

### (5-(3-{trifluoromethoxy}phenyl)-9-{1-[4-(trifluoromethyl)phenyl]propyl}-2,3,4,9-tetrahydro-1H-carbazol-1-yl)acetic acid

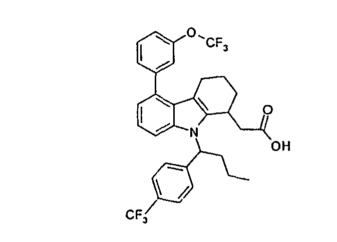

The intermediate from Example 3c, Step 2 (733mg) was dissolved in THF (80ml) under nitrogen and 3-trifluoromethoxyphenyl boronic acid (383mg) was added followed by potassium carbonate (290mg) as a solution in water (18ml). Finally, Pd(PPh₃)₄ (78mg) was added and the reaction heated at 85°C for 15hrs. Water was added and the mixture extracted with ethyl acetate. The organic phase was washed with brine, evaporated and purified by chromatography (silica gel / 20:1 hexane : ethyl acetate) to yield the desired ester (700mg) which was hydrolysed using the procedure of example 1, step 3 to afford the desired (5-(3-{trifluoromethoxy}phenyl)-9-{1-[4-(trifluoromethyl)phenyl] propyl}-2,3,4,9-tetrahydro-1H -carbazol-1-yl)acetic acid which was separated into single isomers by supercritical fluid chromatography. Diastereoisomer A: ¹H NMR δ ppm (CDCl₃) 7.57 (2H, d, J=8.3Hz), 7.37-7.45 (2H, m), 7.38 (2H, d, J=8.3), 7.23 (1H, d, J=8), 6.98 (1H, t, J=7.5Hz), 6.91 (1H, d, J=6.7Hz), 6.84 (1H, d, J=8Hz), 5.48 (1H, dd, J=3.5,11Hz), 3.51 (1H, d, J=9.5Hz), 1.50-2.60 (13H, m), 0.91 (3H, d, J=7Hz). Diastereoisomer B: ¹H NMR δ ppm (CDCl₃) 7.42-7.65 (5H, m), 7.15-7.40 (3H, m), 7.06 (1H, t, J=9Hz), 6.89 (1H, d, J=9Hz), 5.49 (1H, br s), 3.45 (1H, br s), 1.25-2.80 (13H, m) 1.02 (3H, t, J=9Hz) Following analogous procedures, using the appropriate boronic acid and using the intermediate from Step 2 of Example 3a, 3b or 3c as appropriate, the following were prepared:

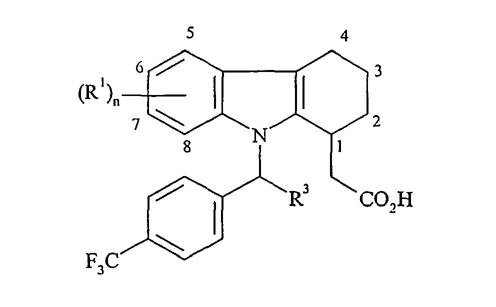

| **Example** | **(R¹)ₙ** | **R³** | **mass spec** |
|---|---|---|---|
| 9a | 5-(3,4-di-Cl-Ph) | n-propyl | 574 |
| 9b | 5-(3,4-di-Cl-Ph) | ethyl | 560 |
| 9c | 5-(3,4-di-Cl-Ph) | H | 532 |
| 9d | 5-(3-MeS-Ph) | H | 510 |
| 9e | 6-(3,4-di-Cl-Ph) | n-propyl | 574 |
| 9f | 5-(2,5-di-Me-Ph) | H | 492 |
| 9g | 7-(3,4-di-Cl-Ph) | H | 530 (M-H) |
| 9h | 7-(3-pyridyl) | H | 465 |
| 9i | 7-(3-thienyl) | H | - |
| 9j | 7-(2,5-di-Me-Ph) | H | 492 |

### EXAMPLE 10

### (6-Trifluoromethyl-9-{4-(trifluoromethyl)benzyl}-2,3,4,9-tetrahydro-1H -carbazol-1-yl)acetic acid

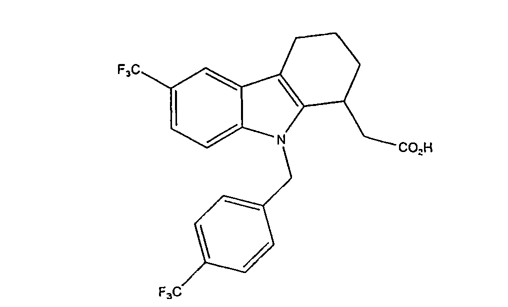

To a mixture of iodine (3.9 g, 15.5 mmol) and silver sulfate (4.8 g, 15.5 mmol) in ethanol (150 ml) was added 4-trifluoromethylaniline (2.5 g, 15.5 mmol). The mixture was stirred for 2 h, filtered, the solids washed well with EtOAc and the filtrate concentrated *in vacuo.* The residue was take up in DCM (100 ml) washed with 5% aqueous sodium hydroxide (50 ml), water (40 ml), brine (30 ml), dried (MgSO₄), filtered and evaporated. The crude 2-iodo-4-trifluoromethylaniline was purified by flash chromatography eluting with 4:1 *ⁱ*hexane/DCM to give an orange solid (3.1 g). To the 2-iodo-4-trifluoromethylaniline from the foregoing step (900 mg, 3.14 mmol) in dry DMF (2 ml) were added ethyl 2-cyclohexanoneacetate (0.62 ml, 3. 5 mmol), p-toluenesulfonic acid (20 mg), and tetraethoxysilane (0.9 ml, 4.1 mmol). The mixture was heated at 130°C for 5 h, allowed to cool to 110°C and more DMF (5 ml) was added followed by Hunig's base (2 ml) and palladium acetate (30 mg, 5 mol%). Reaction heated at 110°C for 15 h. After cooling, the reaction was diluted with EtOAc (100 ml), washed with water (2 x 30 ml), 2M aqueous HCl (30 ml), brine (30 ml), dried (MgSO₄), filtered and evaporated. The crude ethyl ({6-trifluoromethyl}-2,3,4,9-tetrahydro-1-carbazol-1-yl)acetate was purified by flash chromatography eluting with *ⁱ*hexane to 10:1 *ⁱ*hexane/ether to give a yellow oil (260 mg) which was benzylated under the conditions of Example 1, Step 2. The resultant ethyl (6-trifluoromethyl-9-{4-(trifluoromethyl)benzyl}-2,3,4,9-tetrahydro-1H-carbazol-1-yl)acetate was hydrolysed using the procedure of Example 1, Step 3 to give the desired (6-trifluoromethyl-9-{4-(trifluoromethyl)benzyl}-2,3,4,9-tetrahydro-1H -carbazol-1-yl)acetic acid a white solid (40 mg).
¹H NMR (MeOD) 7.76 (1H, s), 7.55 (2H, d, J = 8.2 Hz), 7.32 (2H, m), 7.07 (2H, d, J = 8.2 Hz), 5.58 (1H, d, J=17.7 Hz,), 5.48 (1H, d, J= 17.7 Hz), 3.31 (1H, m), 2.92-2.81 (1H, m), 2.72-2.62 (1H, m), 2.48 (2H, m), 1.99-1.81 (4H, m).
m/z = 456 [MH]⁺

### EXAMPLE 11

### (6-isopropyl-9-{2-methoxy-1-[4-(trifluoromethyl)phenyl]ethyl}-2,3,4,9-tetrahydro-1H-carbazol-1-yl)acetic acid

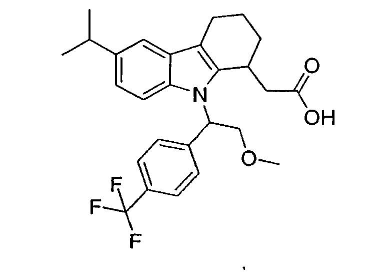

### Step 1 2-bromo-2-[4-(trifluoromethyl)phenyl]ethanol

[4-(trifluoromethyl)phenyl]oxirane (980 mg, 5.27 mmol) (prepared according to J. Med. Chem. 2002, 45 (18), 3891) was dissolved in CHCl₃ (20 ml), HBr (48% aq, 15 ml) was added and the mixture stirred at room temperature for 3 hours. The organic layer was separated, washed with NaHCO₃ solution (20 ml) and with brine (20 ml), dried over MgSO₄ and concentrated *in vacuo* to yield 1.2 g (86%) of the title compound: δ_{H} (360 MHz, CDCl₃) 7.64 (2 H, d, J = 8.3 Hz), 7.56 (2 H, d, J = 8.3 Hz), 5.07 (1 H, dd, J = 5.8, 7.4 Hz), 4.12-3.96 (2 H, m), 2.13 (1 H, m).

### Step 2 2-[1-(4-isopropylphenyl)hydrazino]-2-[4-(trifluoromethyl) phenyl]ethanol

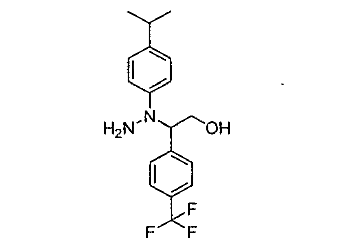

(4-Isopropylphenyl)hydrazine hydrochloride (450 mg, 2.4 mmol) was suspended in toluene (10 ml), Et₃N (0.43 ml, 3.1 mmol) was added and the mixture refluxed for 1 hour and then cooled to room temperature. 2-Bromo-2-[4-(trifluoromethyl)phenyl]ethanol from the foregoing step (0.48 g, 1.8 mmol) in toluene (2 ml) was added and the solution was heated to 80°C for 5 hours. After cooling to room temperature the white solid formed during the reaction was filtered and the solution concentrated. Purification by chromatography on silica gel eluting with a gradient 10-50% ethyl acetate/ hexane afforded 330 mg of the title compound (40%): δ (360 MHz, CDCl₃): 7.55 (2 H, d, J = 8.3 Hz), 7.38 (2 H, d, J = 8.3 Hz), 7.13-7.09 (2 H, m), 6.86-6.82 (2 H, m), 4.82 (1 H, dd, J = 3.4, 7.8 Hz), 4.27 (1 H, dd, J = 7.8, 11.5 Hz), 4.01 (1 H, dd, J = 3.4, 11.5 Hz), 3.73 (2 H, s), 2.85-2.77 (1 H, septet, J = 6.9), 1.19 (6 H, d, J = 6.9), *m*/*z* (ES⁺) 339 (MH⁺), 322 (M-NH₂⁺).

### Step 3 Ethyl (9-{2-hydroxy-1-[4-(trifluoromethyl)phenyl]ethyl}-6-isopropyl-2,3,4,9-tetrahydro-1H-carbazol-1-yl)acetate

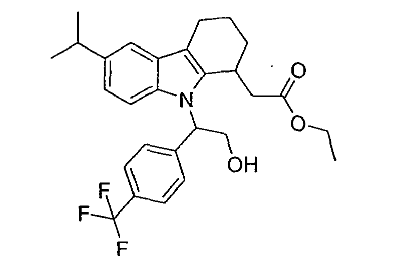

2-[1-(4-Isopropylphenyl)hydrazine]-2-[4-(trifluoromethyl) phenyl] ethanol from the foregoing step (1.0 g, 2.96 mmol) was dissolved in ethanol (20 ml) and ethyl (2-oxocyclohexyl)acetate (0.53 ml, 2.96 mmol) and p-toluenesulphonic acid monohydrate (1.12 g, 5.97 mmol) added and the reaction mixture refluxed under nitrogen overnight. The solvent was then concentrated *in vacuo* and the residue was purified by chromatography on silica gel eluting with 20% ethyl acetate/ hexane to give 970 mg of the title compound (67%) as a 1:1 mixture of diastereoisomers: *m*/*z* (ES⁺) 488 (MH⁺).

### Step 4 Ethyl (6-isopropyl-9-{2-methoxy-1-[4-(trifluoromethyl)phenyl]ethyl}-2,3,4,9-tetrahydro-1H-carbazol-1-yl)acetate

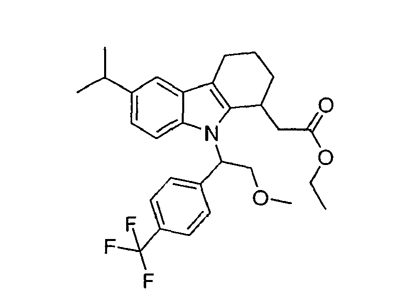

Ethyl (9-{2-hydroxy-1-[4-(trifluoromethyl)phenyl]ethyl}-6-isopropyl-2,3,4,9-tetrahydro-1H -carbazol-1-yl)acetate from the foregoing step (100 mg, 0.2 mmol; 1:1 mixture of diasteroisomers) was dissolved in DMF (5 ml) and cooled to 0°C. NaH (60% dispersion in oil, 9 mg, 0.2 mmol) was added and the mixture stirred at 0°C for 30 minutes. Then MeI (38 µl, 0.6 mmol) was added and the mixture slowly warmed to room temperature. After stirring for 30 minutes at room temperature, H₂O (10 ml) and ethyl acetate (20 ml) were added, the layers separated and the organic dried over MgSO₄ and concentrated *in vacuo.* Purification by chromatography on silica gel eluting with 50% ethyl acetate/ hexane afforded 47 mg of the title compound (47%) as a 3:2 mixture of diasteroisomers.

### Step 5 (6-isopropyl-9-{2-methoxy-1-[4-(trifluoro methyl)phenyl]ethyl}-2,3,4,9-tetrahydro-1H-carbazol-1-yl)acetic acid

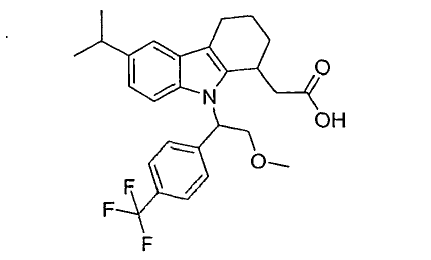

Ethyl (6-isopropyl-9-{2-methoxy-1-[4-(trifluoromethyl)phenyl]ethyl}-2,3,4,9-tetrahydro-1H-carbazol-1-yl)acetate from the foregoing step (46 mg, 0.092 mmol; 3:2 mixture of diasteroisomers) was dissolved in THF (2 ml) and LiOH (22 mg, 0.92 mmol) in H₂O (1 ml) added. The reaction mixture was stirred heating to 50°C for 12 hours. It was then diluted with ethyl acetate (20 ml), washed with 1 N HCl (20 ml), brine (20 ml), dried over MgSO₄ and concentrated *in vacuo.* Purification by chromatography on silica gel eluting with 50% ethyl acetate/ hexane and then with 50% ethyl acetate/ hexane + 0.1% acetic acid followed by a further purification with preparative HPLC (gradient 30-85% CH₃CN-0.1%TFA/H₂O) afforded 27 mg of the desired (6-isopropyl-9-{2-methoxy-1-[4-(trifluoromethyl)phenyl]ethyl}-2,3,4,9-tetrahydro-1H-carbazol-1-yl)acetic acid (67%) as a 1:1 mixture of diasteroisomers (a+b): δ (360 MHz, CDCl₃) 7.56 (1 H, d, J = 8.2 Hz, a/b), 7.50 (1 H, d, J = 8.1 Hz, a/b), 7.41 (1 H, d, J = 8.2 Hz, a/b), 7.32 (1 H, m, a+b), 7.20 (1 H, d, J = 8.2 Hz, a/b), 6.86 (1.5 H, m, a+b), 6.77 (0.5 H, d, J = 8.5 Hz, a/b), 5.66-5.61 (1 H, m, a+b), 4.44 (0.5 H, dd, J = 6.0, 9.7 Hz, a/b), 4.33-4.25 (1 H, m, a/b), 3.97 (0.5 H, dd, J = 6.2, 9.8 Hz, a/b), 3.55 (0.5 H, br d, a/b), 3.45 (0.5 H, br d, a/b), 3.37 (1.5 H, s, a/b), 3.31 (1.5 H, s, a/b), 3.05-2.84 (2.5 H, m, a+b), 2.72-2.39 (2.5H, m, a+b), 1.99-1.85 (4H, m, a+b) 1.27 (6 H, dd, J = 4.1, 6.8 Hz, a+b); *m*/*z* (ES⁺) 474 (MH⁺).

### EXAMPLE 12

### (6-Isopropyl-4,4-dimethyl-9-{1-[4-(trifluoromethyl)phenyl]propyl}-2,3,4,9-tetrahydro-1H-carbazol-1-yl)acetic acid

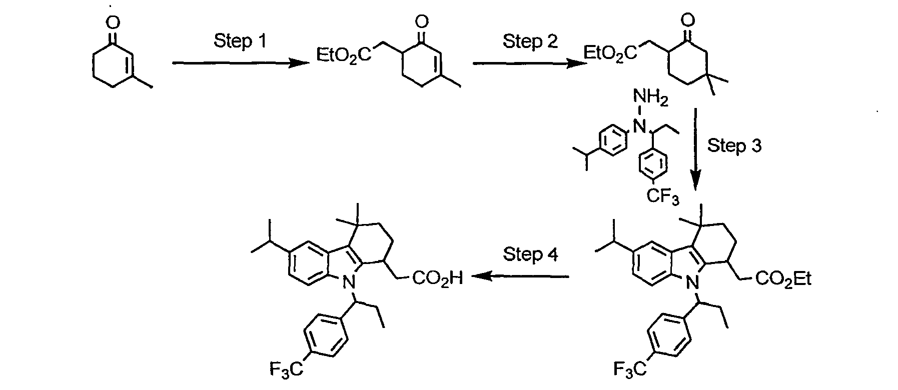

### Step 1 Ethyl (4-methyl-2-oxocyclohex-3-en-1-yl)acetate

A solution of 3-methyl-2-cyclohexen-1-one (3.0g, 27.23mmol) in THF (10ml) was added dropwise into a stirring solution of LDA (1.5M in THF, 19.97ml) in THF (10ml) at -78°C. After stirring for 30min at -78°C, a solution of ethyl bromoacetate (3.34m, 29.96mmol) in THF (10ml) was added dropwise. After addition, stirring was continued at ambient temperature for 2hrs. The reaction mixture was quenched with HCl (2N, 100ml) and the mixture was extracted with ethyl acetate (2×100ml). The organic extract was washed with brine (1×100ml), dried over MgSO₄ and concentrated in vacuo. The residue was purified by flash chromatography eluting with 20% ethyl acetate in hexane to afford (4-methyl-2-oxocyclohex-3-en-1-yl)acetate as a yellow oil (3.76g, 70%).1H NMR δ (ppm)(360MHz, CDCl3): 5.88 (1 H, s), 4.19-4.11 (2 H, m), 2.89 (1 H, dd, J = 5.3, 16.2 Hz), 2.79-2.71 (1 H, m), 2.46-2.22 (3 H, m), 2.14-2.04 (1 H, m), 1.96 (3 H, s), 1.84-1.76 (1 H, m), 1.32-1.24 (3 H, m).

### Step 2 Ethyl (4,4-dimethyl-2-oxocyclohexyl)acetate

Copper iodide (225mg, 1.18mmol) was added into a solution of methylmagnesium iodide (3.39ml, 3M in THF, 10.19mmol) in ether (10ml) at -5°C. The resulting mixture was stirred for 30 min and then enone from the foregoing step (2.0g, 10.19mmol) in ether (10ml) was added dropwise. The reaction mixture was stirred at - 5°C for 2hrs and then at ambient temperature for 1hr. The reaction mixture was quenched with sat. ammonium chloride (100ml) and extracted with ether (3×100ml). The organic extract was washed with brine (1×100ml), dried over MgSO₄ and concentrated in vacuo. The residue was purified by gradient flash chromatography eluting with 1% to 2% ethyl acetate in hexane to afford ethyl (4,4-dimethyl-2-oxocyclohexyl)acetate as an oil (695mg, 32%).1H NMR δ (ppm)(400MHz, CDC13): 4.16-4.09 (2 H, m), 2.80-2.70 (2 H, m), 2.30 (1 H, d, J = 13Hz), 2.18-2.11 (2 H, m), 2.05-1.99 (1 H, m), 1.74-1.55 (3 H, m), 1.26 (3 H, t, J = 7.1Hz), 1.08 (3 H, s), 0.87 (3 H, s).

### Step 3 Ethyl (6-isopropyl-4 4-dimethyl-9-{1-[4-(trifluoromethyl) phenyl]propyl}-2,3,4,9-tetrahydro-1H-carbazol-1yl)acetate

A mixture of ethyl (4,4-dimethyl-2-oxocyclohexyl)acetate from the foregoing step (200mg, 0.94mmol), 1-(4-isopropylphenyl)-1-{1-[4-(trifluoromethyl)phenyl]propyl} hydrazine (prepared from ethyl magnesium chloride and 4-trifluoromethylbenzaldehyde using the procedure of Example 13, Step 1 followed by treatment with carbon tetrabromide/triphenyl phosphine using the procedure of Example 13, Step 4 and finally treatment with 4-isopropylphenyl hydrazine hydrochloride using the procedure of Example 13, Step 5) (317mg, 0.94mmol) and PTSA (360mg, 1.88mmol) in ethanol (30ml) was refluxed for 18hrs. The reaction mixture was cooled to ambient temperature and concentrated in vacuo. The residue was diluted with ethyl acetate (100ml) and sequentially washed with water (1× 100ml), hydrochloric acid (2N, 100ml) and brine (100ml). The organic extract was dried over MgSO₄, concentrated in vacuo, and purified by flash chromatography eluting with 2% ethyl acetate in hexane to afford the desired product (ethyl (6-isopropyl-4,4-dimethyl-9- {1-[4-(trifluoromethyl)phenyl]propyl}-2,3,4,9-tetrahydro-1 H -carbazol-1- yl)acetate) as a mixture of two diastereoisomers A & B (2:1) (112mg, 23%). 1H NMR (360MHz, CDCl₃) δ: 7.54-7.42 (8 H, m, diast A+B), 7.20-7.18 (1 H, m, diast A), 7.12-7.08 (1 H, m, diast B), 7.02-6.88 (1 H, m, diast A), 6.90-6.88 (1 H, m, diast B) 6.82-6.78 (1 H, m, diast A), 6.70-6.67 (1 H, m, diast B), 5.42-5.34 (2 H, m, diast A+B), 4.19-4.08 (4 H, m, diast A+B), 3.52-3.44 (1 H, m, diast B), 3.38-3.32 (1 H, m, diast A) 3.06-2.92 (2 H, m, diast A+B), 2.71-2.42 (8 H, m, diast A+B), 1.88-1.72 (4 H, m, diast A+B), 1.64-1.52 (8 H, m, diast A+B), 1.40-1.02 (20 H, m, diast A+B), 0.90-0.80 (12 H, m, diast A+B) ppm

### Step 4 (6-Isopropyl-4,4-dimethyl-9-{1-[4-(trifluoromethyl)phenyl]propyl}-2,3,4,9-tetrahydro-1H-carbazol-1-yl)acetic acid

A solution of lithium hydroxide (52mg, 2.2mmol) in water (2ml) was added into a solution of ethyl (6-isopropyl-4,4-dimethyl-9-{1-[4-(trifluoromethyl)phenyl]propyl}-2,3,4,9-tetrahydro-1H -carbazol-1-yl)acetate from the foregoing step (110mg, 0.22mmol) in dioxane (4ml) and stirred at 60°C for 18hrs. The reaction mixture was diluted with hydrochloric acid (2N, 20ml) and extracted with ethyl acetate (2×50ml). The organic extract was washed with brine (1×50ml), dried over MgSO₄ and concentrated in vacuo, to afford (6-isopropyl-4,4-dimethyl-9-{1-[4-(trifluoromethyl) phenyl]propyl}-2,3,4,9-tetrahydro-1H -carbazol-1-yl)acetic acid as a mixture of two diastereoisomers A & B (2:1) (10mg, 10%) 1H NMR (400MHz, CDCl₃) δ:7.57-7.50 (4 H, m, diast A+B), 7.35 (1 H, d, J = 8.0Hz, diast A), 7.18 (1 H, d, J = 8.0Hz, diast A), 7.12 (1 H, d, J = 8.5Hz, diast B), 6.91 (1 H, d, 8.4Hz, diast A), 6.83 (1 H, d, J = 8.5Hz, diast B), 6.70 (1 H, d, J = 8.4Hz, diast B), 5.36-5.30 (2 H, m, diast A+B), 3.49-3.42 (1 H, m, diast B), 3.40-3.31 (1 H, m, diast A), 3.01-2.94 (2 H, m, diast A+B), 2.72-2.41 (8 H, m, diast A+B), 1.86-1.80 (4 H, m, diast A+B), 1.54-1.58 (2 H, m, diast A+B), 1.54-1.52 (6 H, m, diast A+B), 1.36-1.41 (6 H, m, diast A+B)1.22-1.29 (12 H, m, diast A+B), 1.01-1.08 (4 H, m, diast A+B), 0.82-0.91 (6 H, m, diast A+B) ppm.

### EXAMPLE 13

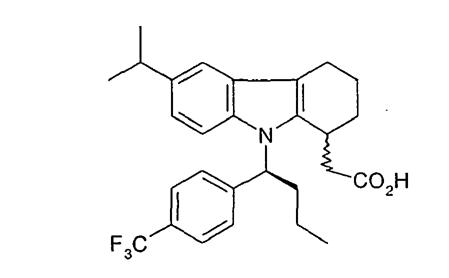

### Step 1

To a solution of 4-trifluoromethylbenzaldehyde (9.7g, 56mmol.) in THF (100ml) at 0°C was added n-propyl magnesium chloride (31ml of 2M solution in ether, 61mmol.) over 5mins. At the end of addition, the solution was stirred 10 minutes at 0°C then warmed to rt for 1 hour and quenched by the addition of a saturated aqueous solution of ammonium chloride (50ml). This was extracted with ether (2x100ml) and the combined organics washed with brine, dried (MgSO₄) and evaporated. The residue was purified by chromatography (silica; eluant 5%EtOAc:hexane) to yield (RS)-1-(4-trifluoromethylphenyl)butan-1-ol as a pale yellow oil (4.7g).

### Step 2

The alcohol from the foregoing step (4.7g, 21.4nmmol.) in acetone (100ml) and cooled to 0°C under nitrogen was treated with Jones reagent (7.5ml) (from a stock of CrO₃ [40g] in water [80ml]/conc. H₂SO₄ [20ml]) portionwise over 10 minutes and the resultant green mixture stirred a further 30 minutes at 0°C. The reaction was diluted with water and extracted with ether (2x100ml). The combined organics were washed with water (3x100ml), dried (MgSO₄) and evaporated. The residue was purified by filtration through a plug of silica (eluant hexane) to yield 1-(4-trifluoromethylphenyl)butan-1-one as a colourless liquid (4.1g).

### Step 3

To a pre-cooled (-30°C) solution of borane.dimethylsulfide complex (3.1ml, 32.4mmol.) in toluene (30ml) was added (R)-methyl CBS oxazaborolidine (1M solution in toluene, 3.3ml), 3.3mmol.) and the mixture stirred 15 minutes at -30°C. The ketone from the foregoing step (7.0g, 32.4mmol.) as a solution in a mixture of dichloromethane (30ml) and toluene (30ml) was added dropwise over 30 minutes and the reaction stirred a further 6h at -30°C. The reaction was quenched by the cautious addition of methanol (7ml) and the mixture diluted with ether and 1N HCl (50ml). The organic layer was separated and washed with further 1N HCl (50ml) and brine, dried (MgSO₄) and evaporated. The residual liquid was purified by chromatography (silica; eluent 5%EtOAc:hexane) to yield (S)-1-(4-trifluoromethylphenyl)-butan-1-ol as a colourless oil (5.22g) which solidified on standing. Derivatization of a sample as its Mosher's ester (MTPACl, Et₃N, DMAP, DCM, rt, 30mins) showed the ee to be 91%.

### Step 4

To a stirred solution of (S)-1-(4-trifluoromethylphenyl)-butan-1-ol from the foregoing step (4.65g, 21mmol., 91%ee) in dry dichloromethane (100ml) cooled to 0°C was added a MgSO₄-dried solution of carbon tetrabromide (9.91g, 1.4 eq.) in dry dichloromethane (50ml). Triphenylphosphine (8.38g, 1.5q.) was then added portionwise over 20 minutes maintaining internal temperature below 6°C. The cooling bath was removed at the end of the addition and the reaction stirred for 30 minutes, reduced to ca. half volume in vacuo and the remaining solution applied to a pad of silica gel and eluted with ether. The combined ether fractions containing product were evaporated in vacuo and the residue purified by flash chromatography (silica; eluant hexane) to yield 5.1g (R)-1-(4-trifluoromethylphenyl)-1-bromobutane.

### Step 5

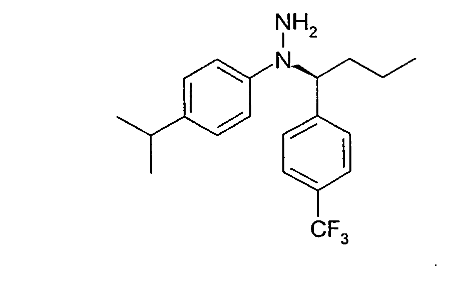

To a stirred suspension of 4-isopropylphenylhydrazine hydrochloride (545mg, 2.8mmol.) in dry THF (20ml) cooled to 0°C was added a solution of NaHMDS (5.9ml, of a 1M solution in THF, 2.1eq.). The cooling bath was removed and the yellow suspension stirred for 1 hour then recooled to 0°C. The bromide from the foregoing step (820mg, 2.9mmol.) as a solution in dry THF (10ml) was added, the cooling bath removed and the mixture stirred at ambient temperature for 17 hours. The reaction was diluted with ether (150ml) and water (100ml) and the layers separated. The aqueous was further extracted with ether (100ml) and the combined ether layers washed with water (2x100ml) and brine (100ml), dried (MgSO₄) and evaporated to give 1.3g of a deep red oil. Purification by column chromatography (silica; eluant 10% ethyl acetate : hexane) gave the product (530mg) as a red oil. (86% ee by chiral HPLC)

### Step 6

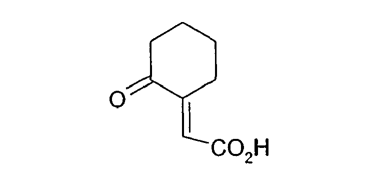

To a solution of sodium methoxide in methanol (25%, 117ml, 0.51mole) at 5°C was added a mixture of cyclohexanone (50g, 1eq.) and diethyl oxalate (70ml, 1eq.) and the mixture stirred at room temperature for 6 hours. The reaction was quenched by the addition of water (1500ml) and EtOAc (1000ml) and the aqueous layer separated and acidified with conc. HCl and extracted with further EtOAc (2x1000ml). The combined organics were dried (MgSO₄) and evaporated in vacuo to give 90g of crude intermediate which was used directly in the next step.
The crude ketoester from the foregoing step was added to a mixture of aqueous potassium dihydrogen phosphate (770ml of 1M solution), aqueous sodium hydrogen phosphate (1700ml of 0.5M solution) and 50% aqueous glyoxylic acid (188ml) at 5°C. The mixture was adjusted to pH 6-7 with conc. NaOH solution then stirred at 5°C for 1 hour before being washed with EtOAc (500ml). The aqueous layer was acidified with conc. HCl then extracted with EtOAc (2x700ml) and the combined organics dried (MgSO₄) and evaporated in vacuo to give a residue which was triturated with heptane to yield the desired product as a pale yellow, gummy solid (37g).

### Step 7 (Fischer indole synthesis)

To a stirred solution of the hydrazine from Step 5 (556mg, 1.59mmol.) in dry isopropanol (20ml) was added p-toluenesulfonic acid monohydrate (271mg, 0.9eq.), the product from Step 6 (489mg, 2eq.) and powdered 3A molecular sieves (600mg) and the resulting mixture heated to gentle reflux for 3 hours. The reaction was cooled, diluted with water (50ml) and extracted into EtOAc (2x100ml). The combined organics were washed with 1N HCl (100ml) and brine (100ml), dried (MgSO₄) and evaporated in vacuo to leave a residue which was purified by chromatography (silica; eluant 1:2 ethyl acetate:hexane) to give the product (460mg) as a yellow foam.

### Step 8 - Asymmetric hydrogenation

To a stirred solution of the product from the foregoing step (460mg, 0.98mmol.) in methanol (25ml) in a thick-walled flask was added triethylamine (0.14m, 1.0eq.) and the solution degassed by nitrogen bubbling for 10 minutes. (S)-Ru(BINAP)Cl₂ (78mg, 10mol%) was added and the mixture placed under an atmosphere of hydrogen (35psi), warmed to 40°C and shaken under hydrogen for 14 hours. The mixture was cooled, diluted with EtOAc (100ml) and washed with 1N HCl (50ml). The aqueous layer was extracted with further EtOAc (100ml) and the combined organics washed with brine (50ml) and dried (MgSO₄). To the resulting yellow solution was added activated charcoal (1g), the suspension stirred 10 minutes and then filtered through a pad of Celite™ washing well with further EtOAc. The filtrate was evaporated to give a residue which was purified by chromatography (silica; eluant 1:2 ethyl acetate:hexane) to give the product (320mg) as a pale brown foam (87:13 mix of diastereomers). This diastereomeric mixture could be crystallized to purity via its dicyclohexylamine salt.
1H NMR (free acid) δ (ppm)(500MHz, CDCl₃): 7.54 (2H, d, J 8.0Hz), 7.33-7.31 (3H, m), 6.87 (1H, dd, J 8.5, 1.5Hz), 6.76 (1H, d, J 8.5Hz), 5.40 (1H, dd, J 10.5, 4.0Hz), 3.48 (1H, m), 2.96 (1H, septet, J 7.0Hz), 2.86 (1H, dd, J = 15.0, 4.0 Hz), 2.68 (1H, m), 2.52-2.44 (2H, m), 2.37-2.30 (2H, m), 1.96 (3H, m), 1.85-1.81 (1H, m), 1.28 (6H, d, J 7.0Hz), 1.21 (1H, m) and 0.87 (4H, m). m/z 472 (MH+).

### EXAMPLE 14

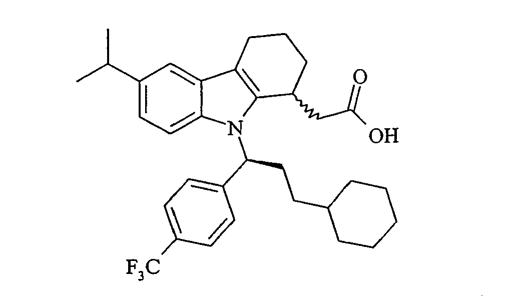

Prepared as described in Example 13, using cyclohexylethylmagnesium bromide in Step 1.

1H NMR δ (ppm)(500 MHz CDCl3,):7.53 (2 H, d, J = 8.2 Hz), 7.34 (1 H, s), 7.31 (2 H, d, J = 8.2 Hz), 6.87 (1 H, d, J = 8.5 Hz), 6.78 (1 H, d, J = 8.5 Hz), 5.35 (1 H, dd, J = 4.4, 10.3Hz), 3.47 (1 H, d, J = 11.4 Hz), 3.00-2.94 (1 H, m), 2.86 (1 H, dd, J = 4.2, 14.9 Hz), 2.72-2.66 (1 H, m), 2.54-2.32 (4 H, m), 2.00-1.92 (3 H, m), 1.88-1.78 (1 H, m), 1.65-1.51 (6 H, m), 1.29 (6 H, d, J = 6.9 Hz), 1.17-1.09 (5 H, m), 0.80-0.77 (2 H, m); m/z (ES+) 540 (MH+).

### EXAMPLE 15

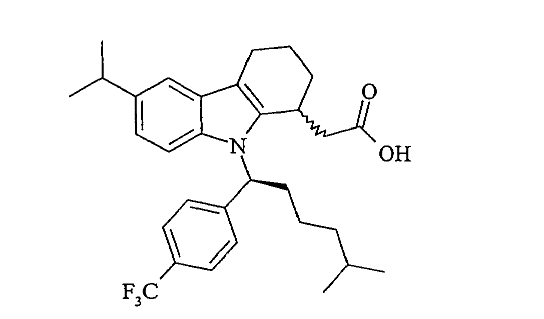

Prepared as described in Example 13, using 4-methylpentylmagnesium bromide in Step 1.

1H NMR δ (ppm)(500 MHz, CDCl3,): 7.54 (2 H, d, J = 8.1 Hz), 7.33-7.31 (3 H, m), 6.87 (1 H, dd, J = 1.3, 4.9 Hz), 6.77 (1 H, d, J = 8.4 Hz), 5.39 (1 H, dd, J = 10.6 and 3.7 Hz), 3.49 (1 H, bd, J = 6.8 Hz), 3.00-2.94 (1 H, m, J = 7.0), 2.86 (1 H, dd, J = 4.6, 15.5 Hz), 2.71-2.64 (1 H, m), 2.55-2.41 (2 H, m), 2.39-2.25 (2 H, m), 2.00-1.92 (3 H, m),1.89-1.77 (1 H, m), 1.43-1.37 (1 H, m), 1.28 (6 H, d, J = 6.9 Hz), 1.23-1.10 (4 H, m), 0.77 (3 H, d, J = 6.6 Hz), 0.71 (3 H, d, J = 6.6 Hz); m/z (ES-) 512 (MH-).

### EXAMPLE 16

### (6-isopropyl-1-propyl-9-{1-[4-(trifluoromethyl)phenyl]butyl}-2,3,4,9-tetrahydro-1H - carbazol-1-yl)acetic acid

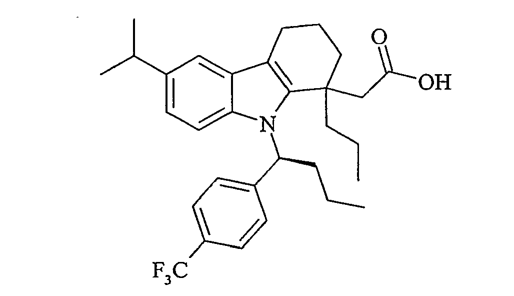

### Step 1 Methyl (2-oxo-1-propylcyclohexyl)acetate

A solution of cyclohexanone (21ml, 200mmol) in THF (30ml) was added dropwise to a stirring solution of LDA (1.5M in THF, 133ml) in THF (100ml) at - 78°C. After stirring for 30min at -78°C a solution of iodopropane (19.5ml, 200mmol) in THF (20ml) was added dropwise and stirring continued at ambient temperature for 14hrs. The reaction was quenched with HCl (2N, 100ml) and the mixture was extracted with ethyl acetate (3×100ml). The combined organic extracts were washed with brine (1×100ml), dried over MgSO₄ and concentrated in vacuo. The residual oil was purified by vacuum distillation (34-36°C/ 10 mmbar) to give 2-propylcyclohexanone as a colourless oil (2.3g, 8%).

A solution of 2-propylcyclohexanone (2.3g, 16.4mmol) in THF (10ml) was added dropwise into a stirring solution of LDA (1.5M in THF, 11ml) in THF (40ml) at -78°C. After stirring for 30min at -78°C, TMSC1 (2.1ml), 16.4mmol) was added dropwise and stirring continued at ambient temperature for 3hrs. The reaction was quenched with water/hexane(200ml/200ml). The organic phase was washed with saturated sodium hydrogen carbonate (1×100ml), dried over MgSO₄ and concentrated in vacuo. The residual oil was purified by vacuum distillation (38-45°C/10mmbar) to give trimethyl[(6-propylcyclohex-1-en-1-yl)oxy]silane as a colourless oil (1.9g, 56%). A solution of potassium-t-butoxide (8.9ml, 1.0M in THF) was added dropwise to a stirring solution of this silane (1.90g, 8.9mmol) in THF (20ml) at -15°C. The reaction mixture was stirred at -15°C for 1hr and then cooled to -78°C. Methyl bromoacetate (0.84ml, 8.9mmol) was added dropwise and stirred at -78°C for 1hr and then at ambient temperature for 10hrs. The reaction mixture was quenched with water (100ml) and extracted with ethyl acetate (2×100ml). The organic extract was washed with brine (100ml), dried over MgSO₄ and concentrated in vacuo. The residual oil was purified by gradient flash chromatography eluting with 2% to 8% ethyl acetate in hexane to give methyl (2-oxo-1-propylcyclohexyl)acetate as a colourless oil (821mg, 43%). 1H NMR (360MHz, CDCl₃) δ: 3.64 (3H, s), 2.45-2.28 (2H, m), 2.01-1.12 (12H, m), 0.91-0.27 (3H, m).

### Step 2 Methyl (6-isopropyl-1-propyl-9-{1-[4-(trifluoromethyl)phenyl]butyl}-2,3,4,9-tetrahydro-1H-carbazol-1-yl)acetate

A mixture of (2-oxo-1-propylcyclohexyl)acetate (821mg, 4.14mmol), 1-(4-isopropylphenyl)-1-{(1*S*)-1-[4-(trifluoromethyl)phenyl]butyl}hydrazine (Example 13 Step 5) (903mg, 2.58mmol) and HCl (2N, 1 drop) in ethanol (30ml) was refluxed for 18hrs. The reaction mixture was cooled to ambient temperature and concentrated. The residue was diluted with ethyl acetate (1 00ml) and sequentially washed with water (1×100ml), hydrochloric acid (2N, 1 00ml) and brine (100ml). The organic extract was dried over MgSO₄ and concentrated in vacuo. The residual oil was purified by flash chromatography eluting with 2% ethyl acetate in hexane to afford the target molecule as a colourless oil of two diastereoisomers A&B(1:1)(112mg, 23%). *m*/*z* (ES⁺) 542 (MH⁺).

### Step 3

The methyl ester from Step 2 was hydrolysed as described in Example 12 Step 4.

Purification by prep HPLC afforded the target compound as a mixture of two diastereoisomers A & B (1:1) (97mg, 34%) 1H NMR (400MHz, CDCl₃) δ: 7.53 (1H, d J = 8Hz , diast A or B), 7.47 (1H, d J = 8Hz , diast A or B), 7.40 (1H, d J = 8Hz , diast A or B), 7.33 (1H, dd J = 8Hz, 2Hz, diast A or B), 7.16-7.09 (1.5H, m, diast A+B), 6.97-6.87 (1.5H, m, diast A+B), 5.72-5.64 (1H, m, diast A+B), 5.34-5.30 (1H, br, diast A+B), 2.92-3.02 (1H, m, diast A+B),1.09-2.88 (21H, m, diast A+B), 1.02-0.83 (4.5H, m, diast A+B), 0.67-0.55 (1H, m, diast A or B) 0.11 (1.5H, t J = 8Hz, diast A or B). *m*/*z* (ES⁺) 512 (MH+).

### EXAMPLE 17

### 6-Isopropyl-9-{1-[4-trifluoromethyl)phenyl]butyl}2,3,4,9-tetrahydrospiro[carbazole-1,1'-cyclopropane]-2'-carboxylic acid

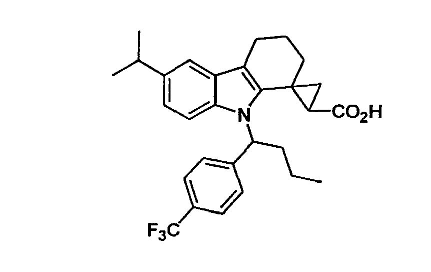

### Step 1

### 4-Oxospiro[2,5]octane-1-carboxylic acid

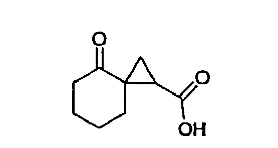

1-Vinylspiro[2,5]octan-4-one (0.16g, 1.06mM) (Roberton, J. et al. Tetrahedron. 2000, 54, 8959-65) was dissolved in dichloromethane: methanol (20ml, 1:1) and cooled to -78°C and ozone bubbled thorough the solution for 5 minutes. Reaction was then stirred for a further 10 minutes before quenching the reaction with dimethylsulfide (1ml) and left to warm to room temperature and stirred overnight. The reaction was evaporated and the crude aldehyde redissolved in tetrahydrofuran, (20ml) treated with sodium chlorite (0.62g, 6.8mM) and sulfamic acid (0.25g, 2.5mM) and stirred for 3 hours at room temperature. The reaction mixture was then partitioned between ethyl acetate and water, the organic layer was washed with brine, dried over magnesium sulphate and purified on silica gel eluting with *ⁱ*hexane-ethyl acetate mixture to give the title compound as a mixture of isomers (0.078g) ms (ES⁺) m/e 168 [MH]⁺. ¹H NMR (250 MHz,CDCl₃) δ 9.5 (1H, br), 2.50-2.45 (1H, m), 2.32-2.20 (1H, m), 2.08-1.97 (2H, m), 1.90-1.78 (4H, m), 1.50-1.44 (1H, m), 1.35-1.10 (1H, m) and 1.04-1.01 (1H, m).

### Step 2

1-(4-Isopropylphenyl)-1-{1-[4-(trifluoromethyl)phenyl]propyl} hydrazine (0.158g, 0.45mM), and 4-oxospiro[2,5]octane-1-carboxylic acid (0.076g, 0.45mM) was dissolved in ethanol (3ml) and treated with p-toluenesulphonic acid (0.154g, 0.81mM) and heated to 80°C for 3 hours. The reaction mixture was evaporated and purified on silica gel eluting with *ⁱ*hexane-ethyl actetate mixture to give the product as a mixture of isomers, 0.021g, ms (ES⁺) m/e 483 [MH]⁺.

## Claims

1. The use, for the manufacture of a medicament for treatment or prevention of a disease associated with the deposition of β-amyloid in the brain, of a compound of formula I: 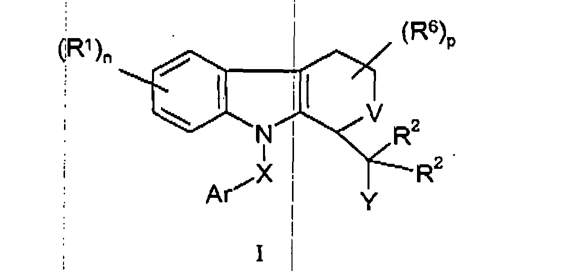
wherein V represents a bond, CH₂ or CH₂CH₂;
X represents CHR³ where R³ is H or a hydrocarbon group containing up to 10 carbon atoms which is optionally substituted with halogen, CF₃, C₁₋₄alkoxy or C₁-₄alkylthio;
Y represents CO₂H;
Ar represents phenyl which optionally bears up to 3 substituents independently selected from hydrocarbon groups of up to 6 carbon atoms and (CH₂)ₘ-Z where m is 0, 1 or 2 and Z represents halogen, N₃, CN₃, CF₃, OCF₃, OR⁴, S(O)ₜR⁴ where t is 0, 1 or 2, CO₂R⁴, tetrazole, N(R⁴)₂, NHCOR⁵, NHCON(R⁴)₂, CON(R⁴)₂, SO₂N(R⁴)₂, NHSO₂R⁵, COR⁵, or OCOR⁵;
n is 0, 1, 2 or 3;
each R¹ is independently selected from nonaromatic hydrocarbon groups of up to 6 carbon atoms and (CH₂)_{q}-W where q is 0, 1 or 2 and W represents halogen, CN, CF₃, OR⁴, N(R⁴)₂, S(O)ₜR⁴ where t is 0, 1 or 2, CO₂R⁴, tetrazole, CON(R⁴)₂, SO₂N(R⁴)₂, COR⁵, OCOR⁵ or phenyl or heteroaryl either of which optionally bears up to 3 substituents selected from halogen, CF₃, OCF₃, CN, OH, C₁₋₄alkyl, C₁₋₄alkoxy, C₁₋₄alkylthio or C₁₋₄alkoxycarbonyl,
each R² is independently H or C₁₋₄alkyl; or one R² group together with an R⁶ group attached at the same ring position as the C(R²)₂-Y moiety completes a spiro-linked hydrocarbon ring of 3-6 members;
R⁴ represents H or a hydrocarbon group of up to 7 carbon atoms, optionally substituted with halogen,: CN, CF₃, OH, C₁₋₄alkoxy or C₁₋₄alkoxycarbonyl; or two R⁴ groups attached to the same nitrogen atom may complete a 5- or 6-membered heterocyclic ring;
R⁵ represents R⁴ that is other than H;
p is 0, 1 or 2; and
R⁶ represents C₁₋₆alkyl, C₂₋₆alkenyl or phenyl, benzyl or heteroaryl, said phenyl, benzyl or heteroaryl optionally bearing up to 3 substituents selected from halogen, CN, CF₃, OCF₃, OR⁴, CO₂R⁴, COR⁵, OCOR⁵ and C₁₋₄alkyl; or an R⁶ group together with an R² group may complete a spiro-linked hydrocarbon ring as defined previously;
or a pharmaceutically acceptable salt thereof; wherein said disease is Alzheimer's disease, cerebral amyloid angiopathy, multi-infarct dementia, dementia pugilistica or Down syndrome.

2. A compound according to formula I as defined in claim 1 wherein p is 1 or 2 and at least one R⁶ represents C₂₋₆ alkenyl or optionally-substituted phenyl, heteroaryl or benzyl;
or a pharmaceutically acceptable salt thereof.

3. A compound according to formula II: 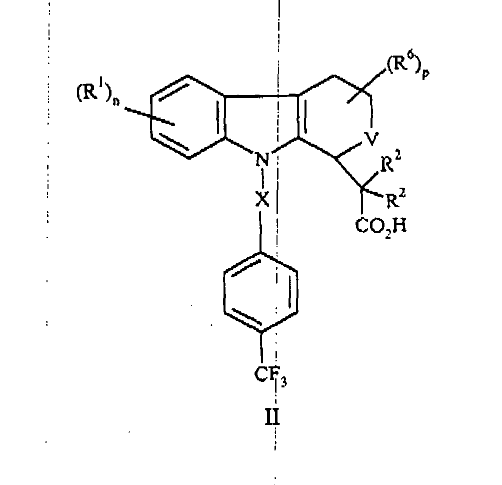 or a pharmaceutically acceptable salt thereof, where V is CH₂ or CH₂CH₂; and X, n, p, R¹, R² and R⁶ are as defined in claim 1;
with the proviso that if V is CH₂, X is CH₂, p is zero and each R² is H, then (R¹)ₙ does not represent 6,8-difluoro.

4. A compound according to formula III: 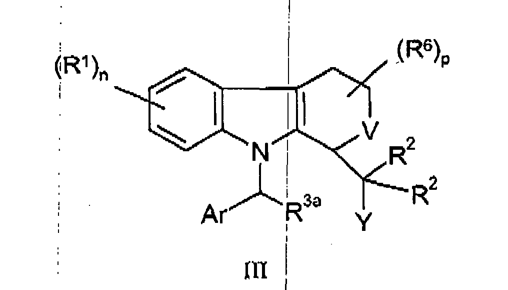 or a pharmaceutically acceptable salt thereof, wherein R^{3a} represents a hydrocarbon group containing from 2 to 10 carbon atoms which is optionally substituted with halogen, CF₃, C₁₋₄alkoxy or C₁₋₄alkylthio; and the remaining variables are as defined in claim with the proviso that R¹ docs not represent SOR⁴ or SO₂R⁴.

5. A compound according to claim 4 wherein Ar represents 4-trifluoromethylphenyl, and both R² groups represent H.

6. A compound according to claim 4 or claim 5 wherein V is CH₂.

7. A compound according to any of claims 4-6 wherein R^{3a} is C₃₋₆alkyl.

8. A compound according to any of claims 2-7 wherein n is 1 or 2 and each R¹ is independently selected from methyl, ethyl, isopropyl, n-butyl, t-butyl, cyclopropyl, Br, Cl, F, CN, CF₃, OCH₃, OCF₃, SCH₃, morpholin-1-yl, 4-fluorophenyl, 3,4-dichlorophenyl, 3-methylthiophenyl, 2,5-dimethylphenyl and 3-trifluoromethoxyphenyl.

9. A compound according to any of claims 2-8 for use in treatment of the human body.

10. A pharmaceutical composition comprising a compound according to any of claims 4-9 and a pharmaceutically acceptable carrier.

11. A process for preparing a compound of formula III as defined in claim 4 comprising the step of hydrogenating a compound of formula (11a) or (11b) over a chiral Ru(BTNAP)Cl₂ catalyst: 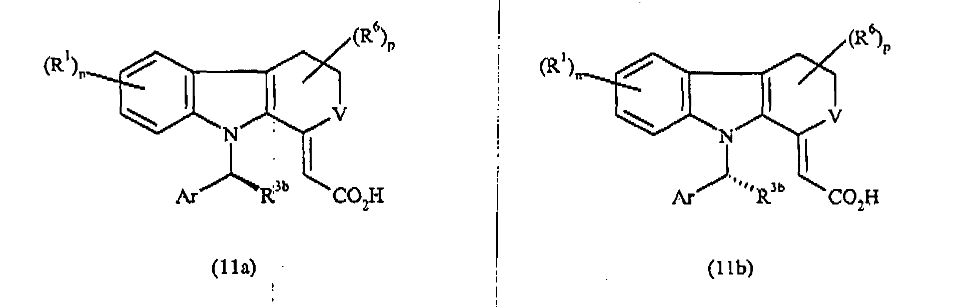 wherein BINAP is bis(diphenylphosphino)-1,1'-binaphthyl and R^{3b} is R³ that is other than H.

12. The process of claim 11 wherein the compound of formula (11a) or (11b) is obtained by reaction of a compound of formula (5a) or (5b) with a compound of formula (10):

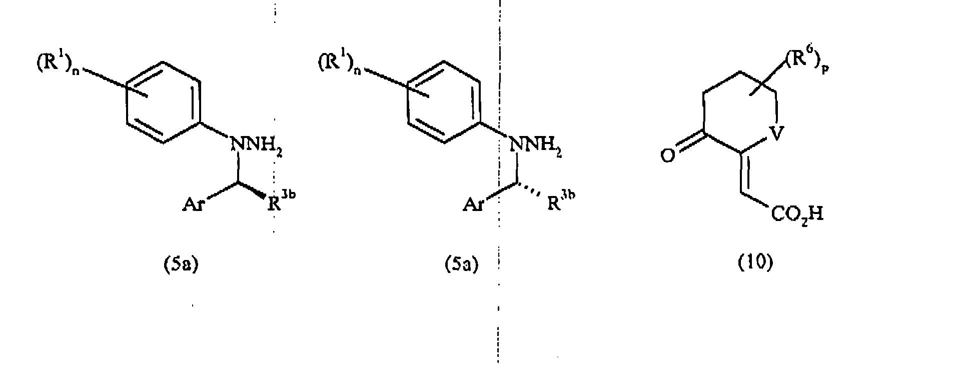

## Patentansprüche

1. Die Verwendung einer Verbindung der Formel I: 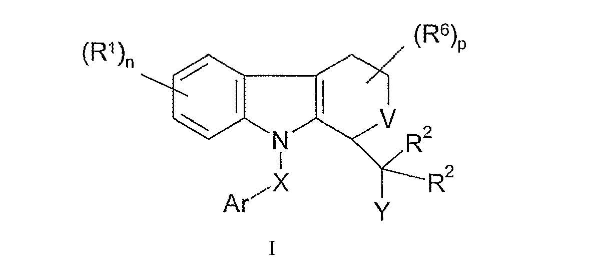 zur Herstellung eines Medikaments zur Behandlung oder Prävention einer Erkrankung, die mit der Ablagerung von β-Amyloid im Gehirn verbunden ist,
wobei V eine Bindung, CH₂ oder CH₂CH₂ bedeutet,
X CHR³ bedeutet, wobei R³ H oder eine Kohlenwasserstoffgruppe mit bis zu 10 Kohlenstoffatomen ist, gegebenenfalls substituiert mit Halogen, CF₃, C₁₋₄-Alkoxy oder C₁₋₄-Alkylthio,
Y CO₂H bedeutet,
Ar Phenyl bedeutet, das gegebenenfalls bis zu 3 Substituenten trägt, unabhängig ausgewählt aus Kohlenwasserstoffgruppen mit bis zu 6 Kohlenstoffatomen und (CH₂)ₘ-Z, wobei m 0, 1 oder 2 ist und Z Halogen, N₃, CN, CF₃, OCF₃, OR⁴, S(O)ₜR⁴, wobei t 0, 1 oder 2 ist, CO₂R⁴, Tetrazol, N(R⁴)₂, NHCOR⁵, NHCON(R⁴)₂, CON(R⁴)₂, SO₂N(R⁴)₂, NHSO₂R⁵, COR⁵ oder OCOR⁵ bedeutet,
n 0, 1,2 oder 3 ist,
jedes R¹ unabhängig ausgewählt ist aus nichtaromatischen Kohlenwasserstoffgruppen mit bis zu 6 Kohlenstoffatomen und (CH₂)_{q}-W, wobei q 0, 1 oder 2 ist und W Halogen, CN, CF₃, OR⁴, N(R⁴)₂, S(O)ₜR⁴, wobei t 0, 1 oder 2 ist, CO₂R⁴, Tetrazol, CON(R⁴)₂, SO₂N(R⁴)₂, COR⁵, OCOR⁵ oder Phenyl oder Heteroaryl, die beide gegebenenfalls bis zu 3 Substituenten tragen, ausgewählt aus Halogen, CF₃, OCF₃, CN, OH, C₁₋₄-Alkyl, C₁₋₄-Alkoxy, C₁₋₄-Alkylthio oder C₁₋₄-Alkoxycarbonyl, bedeutet,
jedes R² unabhängig H oder C₁₋₄-Alkyl ist, oder eine R²-Gruppe zusammen mit einer R⁶-Gruppe, die an die gleiche Ringposition wie der -C(R²)₂-Y-Rest gebunden ist, einen spiroverknüpften Kohlenwasserstoffring mit 3-6 Gliedern vervollständigt,
R⁴ H oder eine Kohlenwasserstoffgruppe mit bis zu 7 Kohlenstoffatomen bedeutet, gegebenenfalls substituiert mit Halogen, CN, CF₃, OH, C₁₋₄-Alkoxy oder C₁₋₄-Alkoxycarbonyl, oder zwei R⁴-Gruppen, die an das gleiche Stickstoffatom gebunden sind, einen 5- oder 6-gliedrigen heterocyclischen Ring vervollständigen können,
R⁵ R⁴ bedeutet, welches kein H ist,
p 0, 1 oder 2 ist und
R⁶ C₁₋₆-Alkyl, C₂₋₆-Alkenyl oder Phenyl, Benzyl oder Heteroaryl bedeutet, wobei Phenyl, Benzyl oder Heteroaryl gegebenenfalls bis zu 3 Substituenten trägt, ausgewählt aus Halogen, CN, CF₃, OCF₃, OR⁴, CO₂R⁴, COR⁵, OCOR⁵ und C₁₋₄-Alkyl, oder eine R⁶-Gruppe zusammen mit einer R²-Gruppe einen wie zuvor definierten spiroverknüpften Kohlenwasserstoffring vervollständigen kann,
oder eines pharmazeutisch annehmbaren Salzes davon, wobei die Erkrankung Alzheimer-Krankheit, zerebrale Amyloidangiopathie, Multiinfarktdemenz, Dementia pugilistica oder Down-Syndrom ist.

2. Eine Verbindung gemäß Formel I wie in Anspruch 1 definiert, wobei p 1 oder 2 ist und wenigstens einer der Reste R⁶ C₂₋₆-Alkenyl oder gegebenenfalls substituiertes Phenyl, Heteroaryl oder Benzyl bedeutet,
oder ein pharmazeutisch annehmbares Salz davon.

3. Eine Verbindung gemäß Formel II: 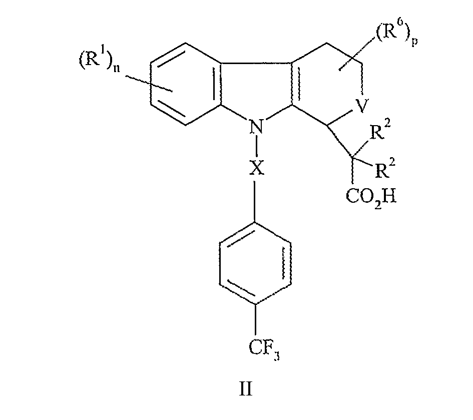 oder ein pharmazeutisch annehmbares Salz davon, wobei V CH₂ oder CH₂CH₂ ist und X, n, p, R¹, R² und R⁶ wie in Anspruch 1 definiert sind,
mit der Maßgabe, dass, wenn V CH₂ ist, X CH₂ ist, p Null ist und jedes R² H ist, (R¹)ₙ dann nicht 6,8-Difluor bedeutet.

4. Eine Verbindung gemäß Formel III: 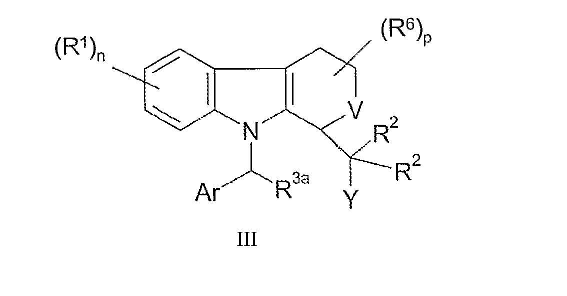 oder ein pharmazeutisch annehmbares Salz davon, wobei R^{3a} eine Kohlenwasserstoffgruppe bedeutet, die 2 bis 10 Kohlenstoffatome enthält, gegebenenfalls substituiert mit Halogen, CF₃, C₁₋₄-Alkoxy oder C₁₋₄-Alkylthio, und wobei die restlichen Variablen wie in Anspruch 1 definiert sind, mit der Maßgabe, dass R¹ nicht SOR⁴ oder SO₂R⁴ bedeutet.

5. Eine Verbindung gemäß Anspruch 4, wobei Ar 4-Trifluormethylphenyl bedeutet und beide R²-Gruppen H bedeuten.

6. Eine Verbindung gemäß Anspruch 4 oder Anspruch 5, wobei V CH₂ ist.

7. Eine Verbindung gemäß einem der Ansprüche 4-6, wobei R^{3a} C₃₋₆-Alkyl ist.

8. Eine Verbindung gemäß einem der Ansprüche 2-7, wobei n 1 oder 2 ist und jedes R¹ unabhängig ausgewählt ist aus Methyl, Ethyl, Isopropyl, n-Butyl, t-Butyl, Cyclopropyl, Br, Cl, F, CN, CF₃, OCH₃, OCF₃, SCH₃, Morpholin-1-yl, 4-Fluorphenyl, 3,4-Dichlorphenyl, 3-Methylthiophenyl, 2,5-Dimethylphenyl und 3-Trifluormethoxyphenyl.

9. Eine Verbindung gemäß einem der Ansprüche 2-8 zur Verwendung bei der Behandlung des menschlichen Körpers.

10. Eine pharmazeutische Zusammensetzung, die eine Verbindung gemäß einem der Ansprüche 4-9 und einen pharmazeutisch annehmbaren Träger umfasst.

11. Ein Verfahren zur Herstellung einer Verbindung der Formel III wie in Anspruch 4 definiert, umfassend den Schritt der Hydrierung einer Verbindung der Formel (11a) oder (11b) über einem chiralen Ru(BINAP)Cl₂-Katalysator: 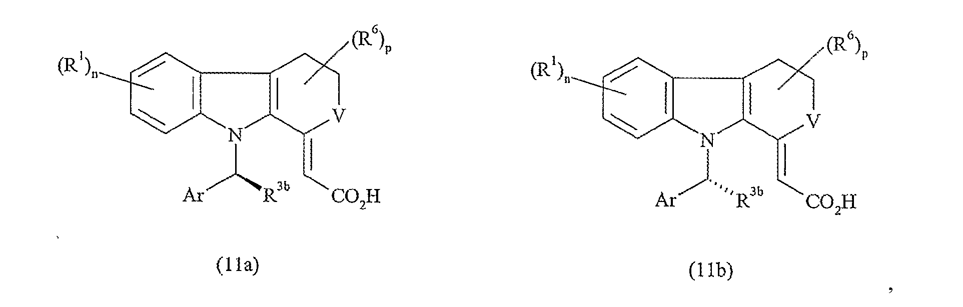 wobei BINAP Bis(diphenylphosphino)-1,1'-binaphthyl ist und R^{3b} R³ ist, das kein H ist.

12. Das Verfahren gemäß Anspruch 11, wobei die Verbindung der Formel (11a) oder (11b) durch Umsetzung einer Verbindung der Formel (5a) oder (5b) mit einer Verbindung der Formel (10) erhalten wird:

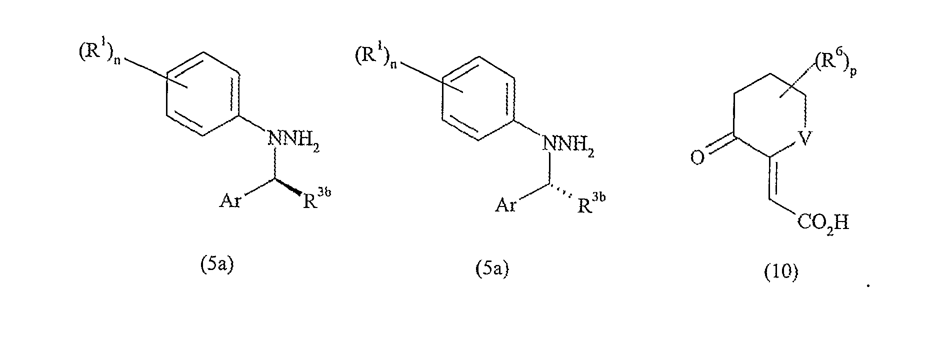

## Revendications

1. Utilisation, pour la fabrication d'un médicament destiné au traitement ou à la prévention d'une maladie associée au dépôt de β-amyloïde dans le cerveau, d'un composé de la formule I: 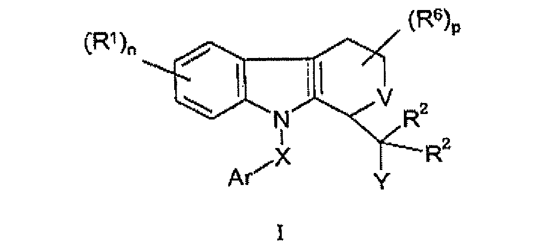
dans laquelle V représente une liaison, CH₂ ou CH₂CH₂;
X représente CHR³ où R³ est H ou un groupe hydrocarboné contenant jusqu'à 10 atomes de carbone qui est éventuellement substitué par des halogènes ou par des groupes CF₃, alcoxy en C₁₋₄ ou alkylthio en C₁₋₄;
Y représente CO₂H;
Ar représente un groupe phényle qui porte éventuellement jusqu'à 3 substituants indépendamment choisis parmi les groupes hydrocarbonés ayant jusqu'à 6 atomes de carbone et (CH₂)ₘ-Z où m vaut 0, 1 ou 2 et Z représente un halogène, N₃, CN, CF₃, OCF₃, OR⁴, S(O)ₜR⁴ où t vaut 0, 1 ou 2, CO₂R⁴, un tétrazole, N(R⁴)₂, NHCOR⁵, NHCON(R⁴)₂, CON(R⁴)₂, SO₂N(R⁴)₂, NHSO₂R⁵, COR⁵, ou OCOR⁵,
n vaut 0, 1, 2 ou 3;
chaque R¹ est indépendamment choisi parmi les groupes hydrocarbonés non aromatiques ayant jusqu'à 6 atomes de carbone et (CH₂)_{q}-W où q vaut 0, 1 ou 2 et W représente un halogène, CN, CF₃, OR⁴, N(R⁴)₂, S(O)ₜR⁴, où t vaut 0, 1 ou 2, CO₂R⁴, un tétrazole, CON(R⁴)₂, SO₂N(R⁴)₂, COR⁵, OCOR⁵ ou un phényle ou un hétéroaryle portant l'un ou l'autre éventuellement jusqu'à 3 substituants choisis parmi les halogènes et les groupes CF₃, OCF₃, CN, OH, alkyle en C₁₋₄, alcoxy en C₁₋₄, alkylthio en C₁₋₄ ou alcoxycarbonyle en C₁₋₄;
chaque R² est indépendamment H ou un groupe alkyle en C₁₋₄; ou un groupe R² conjointement avec un groupe R⁶ attaché sur la même position du cycle que le groupement -C(R²)₂-Y forme un noyau hydrocarboné spiro-lié ayant 3 à 6 chaînons;
R⁴ représente H ou un groupe hydrocarboné ayant jusqu'à 7 atomes de carbone, éventuellement substitué par des halogènes ou par des groupes CN, CF₃, OH, alcoxy en C₁₋₄ ou alcoxycarbonyle en C₁₋₄; ou deux groupes R⁴ attachés au même atome d'azote peut former un noyau hétérocyclique ayant 5 ou 6 chaînons;
R⁵ représente R⁴ qui est différent de H;
p vaut 0, 1 ou 2; et
R⁶ représente un groupe alkyle en C₁₋₆, alcényle en C₂₋₆ ou phényle, benzyle ou hétéroaryle, ledit groupe phényle, benzyle ou hétéroaryle portant éventuellement jusqu'à 3 substituants choisis parmi les halogènes et les groupes CN, CF₃, OCF₃, OR⁴, CO₂R⁴, COR⁵, OCOR⁵ et alkyle en C₁₋₄; ou un groupe R⁶ conjointement avec un groupe R² peut former un noyau hydrocarboné spiro-lié comme défini précédemment;
ou un de ses sels pharmaceutiquement acceptables; où ladite maladie est la maladie d'Alzheimer, l'angiopathie amyloïde cérébrale, la démence vasculaire, la dementia pugilistica ou le syndrome de Down.

2. Composé selon la formule I tel que défini dans la revendication 1 dans lequel p vaut 1 ou 2 et au moins un R⁶ représente un groupe alcényle en C₂₋₆, ou un groupe phényle, hétéroaryle ou benzyle éventuellement substitué;
ou un de ses sels pharmaceutiquement acceptables.

3. Composé selon la formule II: 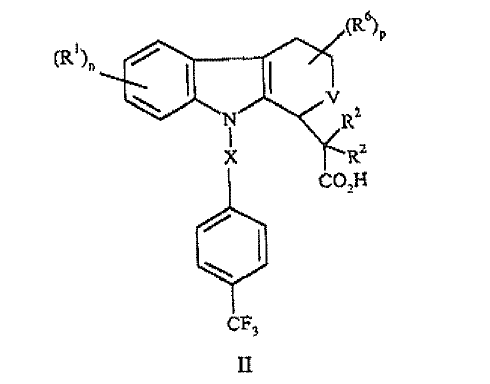
ou un de ses sels pharmaceutiquement acceptables, où V est CH₂ ou CH₂CH₂; et X, n, p, R¹, R² et R⁶ sont tels que définis dans la revendication 1;
sous réserve que si V est CH₂, X est CH₂, p vaut zéro et chaque R² est H, alors (R¹)ₙ ne représente pas un groupe 6,8-difluoro.

4. Composé selon la formule III: 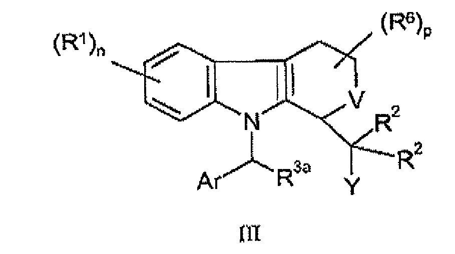 ou un de ses sels pharmaceutiquement acceptables, où R^{3a} représente un groupe hydrocarboné contenant 2 à 10 atomes de carbone qui est éventuellement substitué par des halogènes ou par des groupes CF₃, alcoxy en C₁₋₄ ou alkylthio en C₁₋₄; et les variables restantes sont telles que définies dans la revendication 1, sous réserve que R¹ ne représente pas SOR⁴ ni SO₂R⁴.

5. Composé selon la revendication 4 dans lequel Ar représente un groupe 4-trifluorométhylphényle, et les deux groupes R² représentent chacun H.

6. Composé selon la revendication 4 ou la revendication 5 dans lequel V est CH₂.

7. Composé selon l'une quelconque des revendications 4-6 dans lequel R^{3a} est un groupe alkyle en C₁₋₆.

8. Composé selon l'une quelconque des revendications 2-7 dans lequel n vaut 1 ou 2 et chaque R¹ est indépendamment choisi parmi les groupes méthyle, éthyle, isopropyle, n-butyle, t-butyle, cyclopropyle, Br, Cl, F, CN, CF₃, OCH₃, OCF₃, SCH₃, morpholin-1-yle, 4-fluorophényle, 3,4-dichlorophényle, 3-méthylthiophényle, 2,5-diméthylphényle et 3-trifluorométhoxyphényle.

9. Composé selon l'une quelconque des revendications 2-8 destiné à être utilisé dans le traitement du corps humain.

10. Composition pharmaceutique comprenant un composé selon l'une quelconque des revendications 4-9 et un véhicule pharmaceutiquement acceptable.

11. Procédé de préparation d'un composé de la formule III telle que définie dans la revendication 4 comportant l'étape d'hydrogénation d'un composé de la formule (11a) ou (11b) sur un catalyseur Ru(BINAP)Cl₂ chiral: 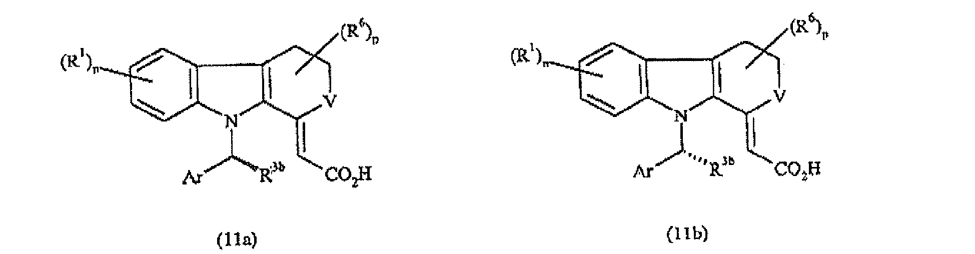 où BINAP est le bis(diphénylphosphino)-1,1'-binaphtyle et R^{3b} est R³ qui est différent de H.

12. Procédé selon la revendication 11 dans lequel le composé de la formule (11a) ou (11b) est obtenu par réaction d'un composé de la formule (5a) ou (5b) avec un composé de la formule (10):
